# EUROPEAN PATENT SPECIFICATION

(11) **EP 2 514 453 B1**
(45) Date of publication and mention of the grant of the patent: **27.11.2013**
(21) Application number: 11163052.1
(22) Date of filing: 19.04.2011
(51) Int. Cl.: A61M 5/172, A61M 5/44, A61F 7/00, A61M 1/36

(54) **Apparatus for controlling a body temperature**
Vorrichtung zur Steuerung der Körpertemperatur
Appareil de contrôle de la température corporelle

(43) Date of publication of application: 24.10.2012
(73) Proprietor: Roth, Matthias, 85376 Giggenhausen (DE); Baenkler, Marc, 91074 Herzogenaurach (DE)
(72) Inventor: Roth, Matthias, 85376 Giggenhausen (DE); Baenkler, Marc, 91074 Herzogenaurach (DE)
(74) Representative: Vossius & Partner

(56) References cited:
- EP-A1- 1 600 186
- WO-A1-2007/078463
- WO-A1-2008/124644
- WO-A2-2009/056640
- US-A1- 2005 107 741

## Description

The present invention relates to an apparatus for controlling the temperature of a patient body, wherein the infusion of a fluid is used for controlling the body temperature.

The positive effect of hypothermia, i.e. the systematical lowering of the body temperature, has been investigated in many studies. The results suggest reduced tissue damage and an improvement of the patient-outcome as a consequence of lowering the body temperature during the acute phase of a patient. The basic mechanism is the general decrease of the reaction rate of (bio-)chemical reactions due to lowered temperatures (van 't Hoff's rule). A typical mode of application may include lowering the body temperature by e.g. 3 [deg.] C and sustaining the lowered temperature over several hours. E.g., induced (i.e. iatrogenic) hypothermia has already become a worldwide standard by evidence based medicine criteria for patients in post-reanimation state. Thanks to the simple application, robustness and effectiveness, successful applications of induced hypothermia may also be expected in connection with the treatment of ischemic apoplexia as animal experiments already suggest. A positive effect can also be expected for acute care of trauma patients. To avoid shivering, medication suppressing the trembling may be administered.

Beside induced hypothermia, deliberate adjustment of the body temperature can be a successful approach of fighting hyperthermic states, such as fever.

Further, there may be other advantageous applications of adjusting body temperature by heating, such as regaining normothermia for a person suffering hypothermia or being subject to cooling therapeutic measures.

The technical means known from the prior art in order to adjust the body temperature of a patient include cooling (or heating) blankets and cooling sleeves. With the devices, known from the prior art, it is difficult or not possible at all to maintain a sufficiently stable body temperature of a patient. For example, surface cooling devices such as fans, particularly cooling blankets and cooling sleeves are not effective if a large percentage of the body area is bandaged. Further, they may hamper other actions performed by medical personnel, for example if the position of the patient needs to be changed or if a body area covered by such a cooling device needs to be accessed. Moreover, surface cooling techniques are generally labour intensive and allow changes in the body temperature at moderate speed.

Endovascular cooling for instance conducted with cooling catheters is also known from the prior art. The heat is transferred from central venous blood through the wall of a central venous catheter to a heat carrier medium inside the catheter. This method is highly invasive and the surface limits its effectiveness due to limited heat transfer area of the catheter. Moreover, the period of application for one catheter is limited as the thrombosis risk will increase over time.

For a more rapid induction of hypothermia, chilled intravenous fluids (e.g., normal saline) are infused. In patients with postanoxic encephalopathy, subarachnoid hemorrhage, or traumatic brain injury, the infusion of large volumes of fluids is generally tolerated well and is considered as safe for patients with acute stroke. The infusion fluids usable for induction of hypothermia are for instance regular isotonic sodium-chloride fluid as well as almost the entire variety of fluids to be infused into emergency and intensive care patients anyway for various medical purposes, such as compensation of fluid loss, providing nutrients, administering pharmaceuticals etc.

One device for the infusion of fluids is described in WO 2009/056640. The document discloses an apparatus and method for adjusting or stabilizing the body temperature of a patient by an actively controlled infusion of fluid of a preferably known and/or controlled temperature, employing a feedback control with body temperature being a measured variable and fluid flow being an actuating variable. According to one embodiment, the adjustment and stabilization of the body temperature of a patient is achieved by an actively controlled balance of volume flows of infusion fluids provided at temperatures differing from one another. The volume flows result in a combined volume flow at a suitable temperature being continuously infused into the patient. The controller of the apparatus controls the infusion of fluid dependent on a variety of input parameters each of them related to a temperature or a flow rate. The temperatures considered are for instance the temperature of the patient, an admissible minimum and maximum temperature of the infusion(s) as well as the ambient temperature. The considered flow rates are for instance the total flow rate of fluid given to a patient or the different flow rates of the different infusions in use. The control unit uses target settings such as for instance a required body temperature and a daily infusion amount entered by a user.

WO 2007/078463 and WO 2008/124644 describe devices for controlling the temperature of a patient by infusion of fluid. US 2005/107741 teaches a device according to the preamble of claim 1.

It is an object of the present invention defined in the appended claims to overcome the drawbacks of the prior art and to provide an improved and/or alternative device for controlling or adjusting a temperature of a patient.

One aspect of the present invention is, for instance, to provide a temperature control device and method for critically ill patients. Another preferred aspect of the present invention is to provide an efficient temperature control preferably allowing relatively quick changes of the body temperature. Yet another preferred object of the present invention is to provide a temperature control, which controls or helps to control the body temperature very precise with preferably minor deviations from a desired target body temperature. Yet another preferred object of the present invention is to provide a temperature control which may be used in a wide temperature range. Yet another preferred object of the present invention is to provide a safe temperature control system, which preferably may be operated without ongoing observation by clinical staff. Another aspect of the present invention is to provide a temperature control which is easy to operate, particularly easy to set up. Yet another preferred object is to provide a temperature control which may be operated and preferably be produced at low costs. The term body relates to a body of a human or an animal.

The object(s) underlying the present invention is(are) particularly solved by the features defined in the independent claims. The dependent claims relate to preferred embodiments of the present invention. Further additional and/or alternative aspects are discussed below.

The temperature of a patient may be controlled, alone or at least inter alia, with a device for controlling or assisting to control a temperature of a patient by an infusion of fluid. The device comprises at least one supply of infusion fluid, at least one body temperature input, at least one additional input, at least one control unit, and at least one actuator. The at least one body temperature input is adapted to receive the actual body temperature Tb of the patient and the at least one additional input adapted to receive at least one additional parameter or value AP representing the actual physiological state of the patient. The at least one control unit communicates with said body temperature input and said additional input. The at least one actuator controls or manipulates or sets or actuates or adjusts at least the actual flow rate FR and/or the actual temperature of the infusion fluid to be infused into the patient in accordance with at least one control signal of said control unit.

The at least one or more additional parameter(s) AP representing the actual physiological state of the patient may be for instance a core vital parameter(s) of the patient in addition to the body temperature of the patient. In other words the at least one additional parameter AP representing the actual physiological state of the patient does not comprise the actual body temperature Tb of the patient.

It may be thereby possible to control the temperature of a patient not only by taking into account the body temperature of the patient but also other parameter(s) of the patient. The body temperature advantageously may thereby be controlled more precisely.

The control unit of the device may control said actuator based on at least a target body temperature Tb, target, the actual temperature Tfa and/or the defined temperature Tfd of the infusion fluid, the actual body temperature Tb, the actual flow rate FR and at least one additional parameter AP representing the actual physiological state of the patient.

Controlling the at least one actuator also comprises for instance manipulating, setting, actuating, adapting and/or adjusting the actuator and thereby the physical infusion parameter(s) of the infusion fluid such as the actual flow rate FR and/or actual temperature of the infusion fluid influenced by the at least one actuator. Thereby the actual body temperature Tb of the patient may be controlled, preferably in a closed loop operation. Representing the actual physiological state of the patient may, for instance, comprise indicating the physiological state of the patient, for instance, by a direct functional relationship between the physiological state of the patient and the at least one additional parameter.

The device may comprise at least one fluid temperature input communicating with the control unit. The at least one fluid temperature input may be adapted to receive the actual temperature Tfa of the infusion fluid. Alternatively or additionally, the device may comprise a defined value for the temperature Tfd of the infusion fluid. The defined value for the temperature of the fluid may be any estimated, preset or determined value derived in a conventional way. The defined value for the temperature of the infusion fluid may for instance be entered by the user.

The device may comprise at least one flow rate input communicating with said control unit. The at least one flow rate input may be adapted to receive the actual flow rate FR of the infusion fluid. The actual flow rate may be provided by any kind of conventional flow sensing device. Alternatively or additionally, the device may, for instance, derive or calculate or estimates the actual flow rate FR of the infusion fluid based on the settings of the actuator. The device may, for instance, relate a certain value for the throttle opening of the actuator to certain flow rate at a certain fluid pressure. The device may, for instance, relate a certain pump motor speed to a certain flow rate at a at a certain fluid pressure. The fluid pressure may be considered as constant. Alternatively, also the pressure may be considered for the determination of the actual flow rate of the infusion fluid.

The control unit may comprise a memory adapted to store data, preferably at least the actual temperature Tfa of the infusion fluid and/or the defined temperature Tfd of the infusion fluid, the actual body temperature Tb, the actual flow rate FR, the at least one additional parameter AP representing the actual physiological state of the patient, at least one parameter defined by the user, and/or pre-defined data set by the manufacturer.

The memory of the device may store all actual and/or historical data available. This may be, for instance, any actual and/or historical data of:
- the flow rate FR, for instance as an accumulated or extrapolated volume flow, for instance measured per 24 hours, per hour, and/or per second;
- the temperature Tfa of the infusion fluid;
- the defined temperature Tfd of the infusion fluid;
- the change rate of body temperature Tb, for instance the cooling rate;
- the body temperature Tb, for instance a profile of the body temperature exemplified as a function body temperature versus time;
- the values of the at least one additional parameter AP;
- any other parameter directly or indirectly impacting the temperature control of a patient such as humidity, room temperature; and/or
- any other actual or historical data.

The memory of the device may store parameter(s) entered by a user and/or manufacturer such as:
a) target or threshold value(s) of infusion parameter(s), like:
   - the maximum and/or minimum flow rate FR, for instance as an accumulated or extrapolated volume flow, for instance measured per 24 hours, per hour, and/or per second;
   - the maximum and/or minimum actual temperature Tfa of the infusion fluid;
   - defined temperature Tfd of the infusion fluid;
   - the maximum and/or minimum fluid pressure;
   - or any other target or threshold value of the infusion;
b) target or threshold value(s) of
   - the maximum and/or minimum body temperature Tb or body temperature profile;
   - the maximum change rate of body temperature Tb, for instance a cooling rate;
c) target or threshold value(s) of additional parameter(s), like:
   - the maximum heart rate variability, the maximum intracranial pressure, the maximum change-rate of intracranial pressure, the maximum intracerebral pressure, and/or the maximum change rate of intracerebral pressure; and/or
d) any other value(s).

The memory of the device may store any other data such as patient specific data, for instance sex, age, etc., or other data related to the temperature control such as date, time, room temperature, humidity, etc.

The control unit may comprise a data processing unit adapted to read the data of said memory and/or of the inputs. For instance the at least one fluid temperature input, the at least one body temperature input, at least one additional input, and/or the at least one flow rate input. The data processing unit may be adapted to process the data to a control signal for said actuator.

The at least one additional parameter AP may be or may comprise an absorbing capacity AC representing the patient's capacity to absorb additional infusion fluid, a reduction rate RR representing the patient's capacity to reduce the infusion fluid in the body, and/or a patient-state-index PSI.

Preferably, at least three additional parameters AP represent the actual physiological state of the patient. The three additional parameters may be the absorbing capacity AC, the reduction rate RR and the patient-state-index PSI. Preferably the three additional parameters may be the amount of extravascular lungwater, the central venous pressure and the change of heart rate.

Preferably, the at least one additional parameter AP or the absorbing capacity AC may be or may comprise at least one or several of the parameters: amount of extravascular lungwater, central venous pressure, intracranial pressure, intraabdominal pressure, compartment pressure, heart rate, heart rate variability, blood pressure, arrhythmia, proBNP-level, ejection fraction of heart, ultrasonic filling status of heart and/or a capillary leak-index representing the value of a capillary leak syndrome.

Preferably, the at least one additional parameter AP or the reduction rate RR may be or may comprise at least one or several of the parameters: glomerular filtration rate, creatinin clearance, creatinin level, urea clearance, urea level, elimination of body waste, and/or elimination rate.

Preferably, the at least one additional parameter AP or the patient-state-index PSI may be or may comprise at least one or several of the patient-state-index parameters PSIP, the patient-state-index parameters PSIP comprising the parameters: peripheral perfusion, splachnik perfusion, glomerular filtration rate, creatinin clearance, creatinin level, creatinin level change rate, urea clearance, urea level, urea level change rate, intracranial pressure, change-rate of intracranial pressure, change-rate of intraabdominal pressure, intraabdominal pressure, change-rate of compartment pressure, compartment pressure, heart rate, heart rate variability, arrhythmia, BNP level, BNP level change rate, NTproBNP-level, NTproBNP level change rate, ejection fraction of heart, ultrasonic filling status of heart, elimination, of body waste and/or elimination change rate.

Preferably, the data processing unit may be adapted to calculate the patient-state-index PSI. The calculation comprises the assessment, prioritization and/or weighting of at least two or several or all patient-state-index parameters PSIP. The calculation could be any mathematical operation including estimations suitable to derive to an assessment and/or weighting.

Preferably, the calculation of the patient-state-index may be, preferably continuously, done by an assessment of the core physiological parameters of the patient, for instance the heart rate and alteration of the heart rate, arterial oxygen saturation, central venous pressure, intracranial pressure, temperature as measured arterial, rectal, dermal, and/or shivering effects.

Preferably, the data processing unit may be adapted to calculate at least one additional parameter AP representing the actual physiological state of the patient, said calculation comprises the assessment, prioritization and/or weighting of at least two of the parameters: absorbing capacity AC, reduction rate RR and/or patient-state-index PSI or parameters thereof. Thus the calculation comprises the assessment, prioritization and/or weighting of at least two of the parameters:
- amount of extravascular lungwater, central venous pressure, intracranial pressure, intraabdominal pressure, compartment pressure, heart rate, heart rate variability, blood pressure, arrhythmia, proBNP-level, ejection fraction of heart, ultrasonic filling status of heart and/or a capillary leak-index representing the value of a capillary leak syndrome;
- glomerular filtration rate, creatinin clearance, creatinin level, urea clearance, urea level, elimination of body waste, and/or elimination rate;
- peripheral perfusion, splachnik perfusion, glomerular filtration rate, creatinin clearance, creatinin level, creatinin level change rate, urea clearance, urea level, urea level change rate, intracranial pressure, change-rate of intracranial pressure, change-rate of intraabdominal pressure, intraabdominal pressure, change-rate of compartment pressure, compartment pressure, heart rate, heart rate variability, arrhythmia, BNP level, BNP level change rate, NTproBNP-level, NTproBNP level change rate, ejection fraction of heart, ultrasonic filling status of heart, elimination of body waste and/or elimination change rate,
- and/or any other parameter representing the actual physiological state of the patient.

For instance, the device may use one additional parameter AP which may be based one or several aforementioned parameters or the device may use several additional parameters AP of aforementioned parameters, either alone or in combination, respectively.

Preferably, said data processing unit may be adapted to calculate at least one additional parameter AP representing the actual physiological state of the patient, said calculation comprises the assessment, prioritization and/or weighting of at least two of the parameters: change of heart rate, central venous pressure and/or amount of extravascular lungwater.

The data processing unit may be adapted to prioritize three additional parameters AP representing the actual physiological state of the patient. For instance, the additional parameters AP amount of extravascular lungwater, central venous pressure and change of heart rate may be prioritized, and the infusion may be interrupted when at least one of said parameters is above a threshold value. Additional parameters AP of second priority may be part of the patient-state-index PSI, and the infusion may be interrupted, when the patient-state-index is above a threshold value.

The user may select and/or prioritize the parameters being an at least one additional parameter AP representing the actual physiological state of the patient and/or being part of the patient-state-index PSI. The user may, for instance, decide which additional parameter AP is a first priority additional parameter AP and which additional parameter AP is of second priority and therefore part of the patient-state-index which thus may be an index representing the state of the second priority parameters. The patient-state-index itself may be considered as having the first priority. The device may interrupt the infusion, if a additional parameter of first priority is above a threshold value.

Alternatively or additionally, the control unit may suggest a selection and/or prioritization, for instance based on an analysis of the actual available patient data. The device may preferably analyze, which additional parameters AP are received by the device from any kind of sensor or medical device. The device may have a default prioritization by the manufacturer, for instance to prioritize extravascular lungwater, central venous pressure and change of heart rate.

Preferably, the control unit may adjust or control via said actuator at least one infusion parameter, preferably the flow rate FR and/or the temperature of the infusion fluid, in accordance with the target body temperature Tb, target, preferably a preset or predefined target body temperature Tb, target, preset and/or a target body temperature profile. For instance, the target body temperature profile may be exemplified as a function of the target body temperature versus time. The actual body temperature Tb of the patient may be controlled in a closed loop operation with a preset target body temperature which varies with time. Preferably, the target body temperature profile may be a user defined profile, preferably based on at least a preset target body temperature Tb, target, preset, a change rate of the body temperature Tb, and/or a target flow rate FR. Preferably, the device calculates and/or suggests a target body temperature profile, preferably based on least a preset target body temperature Tb, target, preset, a change rate of the body temperature Tb, and/or a target flow rate FR. Preferably the target flow rate FR may be a target volume to be infused in 24 hours. Preferably, the target body temperature Tb, target, and/or the target body temperature profile may be adapted to the patient. Preferably, the infusion may be initiated with a maximum flow rate FR and/or a temperature of the fluid which is a minimum temperature in the event of cooling of a patient and a maximum temperature in the event of warming a patient up.

Alternatively or additionally at least one target temperature profile may be preset by the manufacturer. Preferably, the pre-defined data may be stored in the at least one memory and may be accessible for the data processing unit.

Preferably, the control unit may control via said actuator the at least one infusion parameter within at least one upper and/or lower threshold value of the at least one infusion parameter. The threshold value(s) of the at least one infusion parameter may be:
- the maximum and/or minimum flow rate FR, for instance as an accumulated or extrapolated volume flow, for instance measured per 24 hours, per hour, and/or per second;
- the maximum and/or minimum actual temperature Tfa of the fluid to be infused;
- the maximum and/or minimum fluid pressure;
- or any other target or threshold value of the infusion.

Preferably, the control unit may control via said actuator the actual body temperature Tb and/or the change rate of the actual body temperature Tb within at least one upper and/or lower threshold value.

Preferably, the control unit may control via said actuator the at least one infusion parameter in accordance with at least one upper and/or lower threshold value of the at least one additional parameter AP, preferably, in accordance with at least one upper and/or lower threshold value of the absorbing capacity AC, reduction rate RR and/or patient-state-index PSI or parameters thereof, most preferably in accordance with at least one upper and/or lower threshold value of the change of heart rate, central venous pressure and/or amount of extravascular lungwater.

Preferably, the control unit may, preferably automatically, adapt the target body temperature Tb and/or the target temperature profile when the actual target body temperature Tb and/or the target temperature profile cannot be achieved by a setting of the at least one infusion parameter, which is within the upper and/or lower threshold value(s) for the at least one infusion parameter, and which ensures that the at least one additional parameter and/or the body temperature change rate are within the respective upper and/or lower threshold values.

Preferably, the control unit may adapt the target body temperature Tb and/or the target temperature profile when the change rate of the actual body temperature Tb is not within its upper and/or lower threshold value.

Preferably, the at least one target value and/or at least one threshold value for the at least one additional parameter AP, for the at least one infusion parameter, for the actual body temperature Tb, and/or for the change rate of the actual body temperature Tb may be administered by the user and/or may be a pre-defined data set by the manufacturer.

The target value and/or threshold value may be based on the individual assessment of the core vital parameters. Moreover, the device may use and/or suggest the parameters for controlling of a body temperature based on settings of the device manufacturer.

The device may be operated in a manual mode wherein only the target infusion parameters are set by the user and the device infuses the infusion fluid without controlling the infusion parameters in accordance to the actual body temperature.

The system may allow the user, for instance the physician, to realize specific temperature profiles. At each point of time during treatment, the device may be able to assess the state of the patient based on the measured physiological parameters, may match this with the desired temperature profile ― as set by the user ― and adjust the inflow and temperature of the cold infusion. As a consequence, the device may automatically cool-down, hold and rewarm the patient according to the physicians requirements and patients vital parameters.

Further, the device may regulate the inflowing volume and temperature of the infusion to the patient based on at least one vital physiological parameter, a specific patient-state-index, the target values as set by the physician and a preferred temperature profile.

In the event of a target body temperature below the actual body temperature Tb, said control unit is adapted to control said actuator so as to reduce the flow rate FR and to reduce the temperature of the infusion fluid thereby keeping the actual body temperature Tb at the target body temperature when the at least one additional parameter AP, i.e. the change of heart rate, preferably also the reduction rate RR, the absorbing capacity AC and/or the patient-state-index PSI, and most preferably also the central venous pressure and/or amount of extravascular lungwater, is/are above a first upper threshold value, and/or to increase the flow rate FR and to increase the temperature of the infusion fluid thereby keeping the actual body temperature Tb at the target body temperature when the at least one additional parameter AP, i.e. the change of heart rate, preferably also the reduction rate RR, the absorbing capacity AC and/or the patient-state-index PSI, and most preferably also the central venous pressure and/or amount of extravascular lungwater, is/are below a first lower threshold value. The threshold value(s) may vary for each patient.

Preferably, in the event of a target body temperature below the actual body temperature Tb, said control unit may be adapted to control said actuator so as to reduce the flow rate FR and to reduce the temperature of the infusion fluid thereby keeping the actual body temperature Tb at a reduced target body temperature when the at least one additional parameter AP, preferably the reduction rate RR, the absorbing capacity AC and/or a patient-state-index PSI, and most preferably the change of heart rate, central venous pressure and/or amount of extravascular lungwater, is/are above a second upper threshold value. The second upper threshold values may be higher than first threshold values. It may be preferred to have at least a reduced cooling effect compared to a no cooling effect. A reduced target body temperature may be a temperature somewhere between the original target body temperature and the present body temperature.

Preferably, in the event of a target body temperature below the actual body temperature Tb, said control unit may be adapted to stop the supply of infusion to the patient in the event of at least one additional parameter AP, preferably the reduction rate RR, the absorbing capacity AC and/or a patient-state-index PSI, and most preferably the change of heart rate, central venous pressure and/or amount of extravascular lungwater, below a third upper threshold value. The third upper threshold value may be higher than second threshold value.

Preferably, the device may be adapted to trigger an alarm and/or to display an information and/or different status information when the at least one lower and/or upper threshold value is achieved. The device may, for instance display the status of the device and/or patient. Preferably the device may display different content, for instance in different colors depending on present values of the parameters. The device may comprise at least one display, for instance a human-machine-interface such as a touch panel. Besides different visual status information the device may also, additionally or alternatively, provide audible alarms. The alarm could be triggered by any kind of threshold value. Preferably the alarm may be triggered based on a threshold value for the at least one additional parameter AP, for the at least one infusion parameter, for the actual body temperature Tb, and/or for the change rate of the actual body temperature Tb.

Preferably, a first lower threshold value may be, for instance, a central venous pressure of 12 cm H2O, a second lower threshold value may be, for instance, a central venous pressure of 20 cm H2O, and a the third lower threshold value may be, for instance, a central venous pressure of 35 cm H2O. These first, second and third threshold values may be defined differently for each patient. Preferably, the third upper threshold value may be, for instance, a change of heart rate of 20 beats per minute with a heart beat in the range of 60 to 180 beats per minute. The values may be defined differently for each patient.

Preferably, the device may be adapted to trigger an alarm and/or to display an information and/or different status information when the amount of infusion left in said supply may be below a minimum value, and/or the remaining period of time until the infusion needs to be refilled may be below a minimum value. Preferably, the minimum period of time and/or minimum infusion amount may be defined by the user or the manufacturer. Preferably, the remaining period of time may be calculated or estimated by the device based on the remaining infusion amount.

The device may be adapted to allow scalable access, for instance with a patient mode which gives reading access to very few parameters such as the infusion parameters and temperature values, and with an expert mode which access to all parameters of the device. Preferably, the parameters forming the patient-state-index may only be visible in the expert mode.

Preferably, the fluid temperature input, the body temperature input, the additional input, and/or the flow rate input may comprise at least one sensor element, and/or at least one connector for connecting at least one sensor element and/or at least one medical device. The at least one sensor element and/or at least one connector may be any kind of sensor element and/or any kind of connector adapted to receive, measure, and/or process at least the actual temperature Tfa of the infusion fluid, the actual body temperature of the patient Tb, the at least one additional parameter AP representing the actual physiological state of the patient, the actual flow rate FR and/or any other parameter, preferably any one or more of above or below parameter(s). The sensor element may, for instance, be any kind of sensing device such as a temperature or flow rate sensing device. The at least one sensor element may be connected or connectable to the device via conventional electronic cabling or may be configured as a sensor which is part of the device as an inbuilt sensor. The at least one connector of the device may be in electronic communication with a medical device indicating the actual temperature Tfa of the infusion fluid, the actual body temperature of the patient Tb, the at least one additional parameter AP representing the actual physiological state of the patient, the actual flow rate FR and/or any other parameter, preferably any one or more of above or below parameter(s). The medical device may be an intensive care monitoring device.

Therefore, the device may either directly measure core vital parameters, for instance temperature of the patient, or process data, as determined by other intensive care monitors, for instance central venous pressure, in order to assess the general state of the patient represented by the patient-state-index. The device may have an user-interface in order to set the target and/or threshold values based on the requirements, for instance target temperature, preferred temperature profile, tolerable volume, etc.

Preferably, the at least one actuator may be in fluid communication with said supply. Preferably, the supply may be connectable and/or may comprise at least one reservoir. Preferably, the supply may be an inlet connectable to the reservoir, preferably by an inlet tube. The reservoir may preferably be an infusion bag. The reservoir may alternatively be a separate housing, for instance a thermo box. The thermo box may comprise a container for the infusion fluid. Also, an infusion bag may be used as a container in the thermo box. Alternatively, the reservoir may be part of the supply when the reservoir is a part of the device. The supply may be in fluid communication with one or more reservoirs.

Preferably, the reservoir may be connectable and/or comprise at least one temperature regulating device for cooling and/or heating of the infusion of the reservoir. The temperature of at least a part of the infusion, preferably the whole infusion may be regulated by the temperature regulating device. The temperature regulating device may be any kind temperature regulating device adapted for cooling and/or heating of an infusion. The temperature regulating device may be embodied as a cooling cuff for an infusion bag. The temperature regulating device may be integrated into a thermo box. The temperature regulating device may comprise one or more thermoplates, heat exchangers, microwave ovens, cooling units, thermal packs, etc. The temperature regulating device may have a separate control unit regulating the temperature of the reservoir or may be controlled by the control unit of the device. The device may be connectable and/or may comprise at least two reservoirs of infusion fluids. Preferably, the infusion fluids of the at least two reservoirs have different temperatures. Preferably, the temperature of the infusion to be infused may at least partially be influenced by mixing the at least two infusion fluids having different temperatures.

Preferably, the reservoir may be connectable and/or comprise at least one supply temperature sensor. The supply temperature sensor provides preferably a temperature signal to the controller of the temperature regulating device and/or the control unit of the device. Preferably, the temperature regulating device and/or the supply temperature sensor may be in communication with the control unit of the device. Preferably, the supply temperature sensor may serve as sensor element for determining actual temperature Tfa of the infusion fluid. Preferably, the defined temperature Tfd of the infusion fluid may be entered by a user or obtained by an external device, preferably obtained by said temperature regulating device.

In one configuration of the device, no temperature regulating device may be used and the temperature of the infusion fluid may be adapted only prior to infusion by the temperature actuator of the actuator of the device.

In another configuration, only the temperature of the infusion fluid in the reservoir may be controlled by the controller of the temperature regulating device or the control unit of the device via the temperature regulating device to the defined fluid temperature Tfd and only the flow rate FR may be regulated by the actuator of the device.

In yet another configuration, the temperature of the fluid in the reservoir may be controlled by the controller of the temperature regulating device or the control unit of the device via the temperature regulating device to the defined fluid temperature Tfd or any other suitable fluid temperature and the actuator of the device additionally may control the fluid temperature of the infused fluid. The actuator of the device may, moreover, additionally also control the flow rate of the infused fluid. The latter configuration may improve the response time of the body temperature control system.

The device may thus receive two fluid temperature signals, one of the fluid to be infused and one of the stored fluid in the reservoir, and/or may actuate two temperature actuator or temperature regulating device. Such a configuration may have a better control performance than a configuration operating with only one fluid temperature signal and/or only one temperature actuator or temperature regulating device, however, the costs of such a device would also be higher.

Preferably the device may comprise and/or may be connectable to at least one patient connector which may connect the actuator of the device with the patient. The device is thus in fluid communication with the patient. The connector may be an infusion needle connected to a tube.

Preferably the actuator may comprise at least one temperature actuator, preferably at least one heating element, at least one cooling element, and/or at least one fluid mixing element or the like. The temperature actuator may be any kind of actuator adapted to change the temperature of the fluid to be infused. The temperature actuator may be a heat exchanger, microwave and/or a fluid mixing element in fluid communication with two fluids of different temperatures as described in WO 2009/056640.

Moreover, the actuator may comprise at least one flow rate actuator, preferably at least one valve and/or at least one pump. The flow rate actuator may be any kind of actuator adapted to change the flow rate of the fluid to be infused.

Moreover, a method for controlling or assisting to control a temperature of a patient by an infusion of fluid, preferably with aforementioned device for controlling a temperature of a patient by an infusion of fluid, comprises at least the steps of:
- providing the actual body temperature Tb of the patient;
- providing at least one additional parameter AP representing the actual physiological state of the patient;
- controlling at least one actuator which is preferably in fluid communication with a supply of fluid and which controls the actual flow rate FR and/or actual temperature of the infusion fluid to be infused in accordance with at least one control signal of a control unit.

The object(s) underlying the present invention is (are) particularly solved by the following additional and/or alternative aspects discussed below. It is to be noted that the following aspects 1. to 69. do not constitute patent claims but form part of the present description.
1. A device (1) for controlling or assisting to control a temperature of a patient by an infusion of fluid, comprising
   at least one supply (2) of infusion fluid;
   at least one body temperature input (5) adapted to receive the actual body temperature (Tb) of the patient;
   at least one additional input (7) adapted to receive at least one additional parameter (AP) representing the actual physiological state of the patient;
   at least one control unit (10) communicating with said body temperature input (5), and said additional input (7); and
   at least one actuator (13) which controls at least the actual flow rate (FR) and/or actual temperature of the infusion fluid in accordance with at least one control signal of said control unit (10).
2. The device (1) according to aspect 1, wherein said control unit (10) controls said actuator (13) based on at least a target body temperature (Tb, target ), the actual temperature (Tfa) and/or the defined temperature (Tfd) of the infusion fluid, the actual body temperature (Tb), the actual flow rate (FR) and at least one additional parameter (AP) representing the actual physiological state of the patient.
3. The device (1) according to aspect 1 or 2, wherein the device (1) comprises
   at least one fluid temperature input (3) communicating with said control unit (10) and adapted to receive the actual temperature (Tfa) of the infusion fluid, and/or
   a defined value for the temperature (Tfd) of the infusion fluid.
4. The device (1) according to any of the preceding aspects, wherein the device (1) comprises at least one flow rate input (9) communicating with said control unit (10) and adapted to receive the actual flow rate (FR) of the infusion fluid, and/or wherein the device (1) derives the actual flow rate (FR) of the infusion fluid based on the settings of actuator (13).
5. The device (1) according to any of the preceding aspects, wherein said control unit (10) comprises:
   a memory (11) adapted to store data, preferably at least the actual temperature (Tfa) and/or the defined temperature (Tfd) of the infusion fluid, the actual body temperature (Tb), the actual flow rate (FR), the least one additional parameter (AP) representing the actual physiological state of the patient, at least one parameter defined by the user,
   and/or pre-defined data set by the manufacturer, and/or
   a data processing unit (12) adapted to read the data of said memory (11) and/or of said inputs (3, 5, 7, 9) and process the data to a control signal for said actuator (13).
6. The device (1) according to any of the preceding aspects, wherein the at least one additional parameter (AP) comprises:
   an absorbing capacity (AC) representing the patient's capacity to absorb additional infusion fluid,
   a reduction rate (RR) representing the patient's capacity to reduce the infusion fluid in the body, and/or
   a patient-state-index (PSI).
7. The device (1) according to any of the preceding aspects, wherein at least three additional parameters (AP) represent the actual physiological state of the patient, and wherein the three additional parameters are: absorbing capacity (AC), reduction rate (RR) and the patient-state-index (PSI).
8. The device (1) according to any of the preceding aspects, wherein at least three additional parameters (AP) represent the actual physiological state of the patient, and wherein the three additional parameters are: amount of extravascular lungwater, central venous pressure and change of heart rate.
9. The device (1) according to any of the preceding aspects, wherein the at least one additional parameter (AP) or the absorbing capacity (AC) comprises at least one or several of the parameters: amount of extravascular lungwater, central venous pressure, intracranial pressure, intraabdominal pressure, compartment pressure, heart rate, heart rate variability, blood pressure, arrhythmia, proBNP-level, ejection fraction of heart, ultrasonic filling status of heart and/or a capillary leak-index representing the value of a capillary leak syndrome.
10. The device (1) according to any of the preceding aspects, wherein the at least one additional parameter (AP) or the reduction rate (RR) comprises at least one or several of the parameters: glomerular filtration rate, creatinin clearance, creatinin level, urea clearance, urea level, elimination of physiological waste, and/or elimination rate.
11. The device (1) according to any of the preceding aspects, wherein the at least one additional parameter (AP) or the patient-state-index (PSI) comprises at least one or several of the patient-state-index parameter(s) (PSIP), the patient-state-index parameter(s) (PSIP) comprising the parameters: peripheral perfusion, splachnik perfusion, glomerular filtration rate, creatinin clearance, creatinin level, creatinin level change rate, urea clearance, urea level, urea level change rate, intracranial pressure, change-rate of intracranial pressure, change-rate of intraabdominal pressure, intraabdominal pressure, change-rate of compartment pressure, compartment pressure, heart rate, heart rate variability, arrhythmia, BNP level, BNP level change rate, NTproBNP-level, NTproBNP level change rate, ejection fraction of heart, ultrasonic filling status of heart, elimination of physiological waste, and/or elimination change rate.
12. The device (1) according to any of the preceding aspects, wherein said data processing unit (12) is adapted to calculate the patient-state-index (PSI), the calculation comprises the assessment, prioritization and/or weighting of at least two or several or all patient-state-index parameters (PSIP).
13. The device (1) according to any of the preceding aspects, wherein said data processing unit (12) is adapted to calculate at least one additional parameter (AP) representing the actual physiological state of the patient, said calculation comprises the assessment, prioritization and/or weighting of at least two of the parameters: absorbing capacity (AC), reduction rate (RR) and/or patient-state-index (PSI) or parameters thereof.
14. The device (1) according to any of the preceding aspects, wherein said data processing unit (12) is adapted to calculate at least one additional parameter (AP) representing the actual physiological state of the patient, said calculation comprises the assessment, prioritization and/or weighting of at least two of the parameters: change of heart rate, central venous pressure and/or amount of extravascular lungwater.
15. The device (1) according to any of the preceding aspects, wherein said data processing unit (12) is adapted to prioritize three additional parameters (AP) representing the actual physiological state of the patient, wherein the additional parameters (AP) amount of extravascular lungwater, central venous pressure and change of heart rate are prioritized, and wherein the infusion is interrupted when at least one of said additional parameter (AP) is above a threshold value.
16. The device (1) according to any of the preceding aspects, wherein additional parameters (AP) of second priority are part of the patient-state-index (PSI), and wherein the infusion is interrupted when patient-state-index (PSI) is above a threshold value.
17. The device (1) according to any of the preceding aspects, wherein the user selects and/or prioritize the parameters being an at least one additional parameter (AP) representing the actual physiological state of the patient and/or being part of the patient-state-index (PSI).
18. The device (1) according to any of the preceding aspects, wherein said control unit (10) controls via said actuator (13) at least one infusion parameter, preferably the flow rate (FR) and/or the temperature of the infusion fluid, in accordance with the target body temperature (Tb, target ), preferably a preset target body temperature (Tb, target, preset) and/or a target body temperature profile.
19. The device (1) according to aspect 18, wherein the target body temperature profile is a user defined profile, preferably based on at least a preset target body temperature (Tb, target, preset), a change rate of body temperature (Tb), and/or target flow rate (FR).
20. The device (1) according to any of the preceding aspects, wherein said control unit (10) controls via said actuator (13) the at least one infusion parameter within at least one upper and/or lower threshold value of the at least one infusion parameter, and/or controls via said actuator (13) the actual body temperature (Tb) and/or the change rate of the actual body temperature (Tb) within at least one upper and/or lower threshold value.
21. The device (1) according to any of the preceding aspects, wherein said control unit (10) controls via said actuator (13) the at least one infusion parameter in accordance with at least one upper and/or lower threshold value of the at least one additional parameter (AP), preferably of the absorbing capacity (AC), reduction rate (RR) and/or patient-state-index (PSI) or parameters thereof, most preferably at least one upper and/or lower threshold value of the change of heart rate, central venous pressure and/or amount of extravascular lungwater.
22. The device (1) according to any of the preceding aspects, wherein said control unit (10) adapts the target body temperature (Tb) and/or the target temperature profile when the actual target body temperature (Tb) and/or the target temperature profile cannot be achieved by a setting of the at least one infusion parameter, which is within the upper and/or lower threshold value(s) for the at least one infusion parameter, and which ensures that the at least one additional parameter and/or the temperature change rate are within the respective upper and/or lower threshold value(s), and/or when the change rate of the actual body temperature (Tb) is not within its upper and/or lower threshold value.
23. The device (1) according to any of the preceding aspects, wherein at least one target value and/or at least one threshold value for the at least one additional parameter (AP), for the at least one infusion parameter, for the actual body temperature (Tb), and/or for the change rate of the actual body temperature (Tb) is/are administered by the user and/or is/are pre-defined data set by the manufacturer.
24. The device (1) according to any of the preceding aspects, wherein, in the event of a target body temperature below the actual body temperature (Tb), said control unit (10) is adapted to control said actuator (13) so as
   to reduce the flow rate (FR) and to reduce the temperature of the infusion fluid thereby keeping the actual body temperature (Tb) at the target body temperature when the at least one additional parameter (AP), preferably the reduction rate (RR), the absorbing capacity (AC) and/or the patient-state-index (PSI), and most preferably the change of heart rate, central venous pressure and/or amount of extravascular lungwater, is/are above a first upper threshold value,
   and/or
   to increase the flow rate (FR) and to increase the temperature of the infusion fluid thereby keeping the actual body temperature (Tb) at the target body temperature when the at least one additional parameter (AP), preferably the reduction rate (RR), the absorbing capacity (AC) and/or the patient-state-index (PSI), and most preferably the change of heart rate, central venous pressure and/or amount of extravascular lungwater, is/are below a first lower threshold value.
25. The device (1) according to any of the preceding aspects, wherein, in the event of a target body temperature below the actual body temperature (Tb), said control unit (10) is adapted to control said actuator (13) so as
   to reduce the flow rate (FR) and to reduce the temperature of the infusion fluid thereby keeping the actual body temperature (Tb) at a reduced target body temperature when the at least one additional parameter (AP), preferably the reduction rate (RR), the absorbing capacity (AC) and/or a patient-state-index (PSI), and most preferably the change of heart rate, central venous pressure and/or amount of extravascular lungwater, is/are above a second upper threshold value.
26. The device (1) according to any of the preceding aspects, wherein, in the event of a target body temperature below the actual body temperature (Tb), said control unit (10) is adapted to stop the supply of infusion to the patient in the event of at least one additional parameter (AP), preferably the reduction rate (RR), the absorbing capacity (AC) and/or a patient-state-index (PSI), and most preferably the change of heart rate, central venous pressure and/or amount of extravascular lungwater, below a third upper threshold value.
27. The device (1) according to any of the preceding aspects, wherein the device (1) is adapted to trigger an alarm and/or to display an information and/or different status information when the at least one lower and/or upper threshold value is achieved.
28. The device (1) according to any of the preceding aspects, wherein a first lower threshold value is a central venous pressure of 12 cm H2O,
   a second lower threshold value is a central venous pressure of 20 cm H2O,
   a the third lower threshold value is a central venous pressure of 35 cm H2O.
29. The device (1) according to any of the preceding aspects, wherein the third upper threshold value is a change of heart rate of 20 beats per minute with a heart beat in the range of 60 to 180 beats per minute.
30. The device (1) according to any of the preceding aspects, wherein the device (1) is adapted to trigger an alarm and/or to display an information and/or different status information when the amount of infusion left in said supply (1) is below a minimum value, and/or the remaining period of time until the infusion needs to be refilled is below a minimum value.
31. The device (1) according to any of the preceding aspects, wherein the fluid temperature input (3), the body temperature input (5), additional input (7), and/or the flow rate input (9) comprise(s):
   at least one sensor element (4), and/or
   at least one connector (6) for connecting at least one sensor element (4) and/or at least one medical device (8), wherein
   the at least one sensor element (4) and/or at least one connector (6) is/are adapted to receive, measure, and/or process the actual temperature (Tfa) of the infusion fluid, the actual body temperature of the patient (Tb), the at least one additional parameter (AP) representing the actual physiological state of the patient, the actual flow rate (FR) and/or any other parameter.
32. The device (1) according to any of the preceding aspects, wherein said at least one actuator (13) is in fluid communication with said supply (2), and/or wherein said supply (2) is connectable and/or comprises at least one reservoir (17), wherein said reservoir (17) is preferably an infusion bag.
33. The device (1) according to aspect 32, wherein said reservoir (17) is connectable and/or comprises at least one temperature regulating device (18), and/or least one supply temperature sensor (21).
34. The device (1) according to aspect 33, wherein said temperature regulating device (18) and/or said supply temperature sensor (21) is/are in communication with said control unit (10).
35. The device (1) according to any of the preceding aspects, wherein the defined temperature (Tfd) of the infusion fluid is entered by a user or obtained by an external device, preferably obtained by said temperature regulating device (18) and/or said supply temperature sensor (21).
36. The device (1) according to any of the preceding aspects, wherein said device (1) comprises and/or is connectable to at least one patient connector (15) which connects said actuator (13) with the patient.
37. The device (1) according to any of the preceding aspects, wherein said actuator (13) comprises:
   at least one temperature actuator (23), preferably at least one heating element, at least one cooling element, and/or at least one fluid mixing element, and/or
   at least one flow rate actuator (25), preferably at least one valve and/or at least one pump.
38. Method for controlling or assisting to control a temperature, preferably with a device in accordance to any of the preceding aspects, of a patient by infusion of fluid, comprising at least the steps of:
   - providing the actual body temperature (Tb) of the patient;
   - providing at least one additional parameter (AP) representing the actual physiological state of the patient;
   - controlling at least one actuator which controls the actual flow rate (FR) and/or actual temperature of the infusion fluid in accordance with at least one control signal of a control unit.
39. The method according to aspect 38, wherein the controlling further comprises:
   controlling said actuator based on at least a target body temperature (Tb, target ), the actual temperature (Tfa) and/or the defined temperature (Tfd) of the infusion fluid, the actual body temperature (Tb), the actual flow rate (FR) and at least one additional parameter (AP) representing the actual physiological state of the patient.
40. The method according to aspect 38 or 39, further comprising the step of providing the temperature of the infusion fluid, wherein the device (1) preferably comprises at least one fluid temperature input communicating with a control unit and receiving the actual temperature (Tfa) of the infusion fluid, and/or
   wherein the device preferably provides a defined value for the temperature (Tfd) of the infusion fluid.
41. The method according to any of the aspects 38 to 40, further comprising the step of providing the actual flow rate (FR) of the infusion fluid, preferably reciewing from at least one flow rate input, and/or deriving the actual flow rate (FR) of the infusion fluid based on the settings of the actuator.
42. The method according to any of the aspects 38 to 41, further comprising the step of storing data on a memory, preferably at least the actual temperature (Tfa) and/or the defined temperature (Tfd) of the infusion fluid, the actual body temperature (Tb), the actual flow rate (FR), the least one additional parameter (AP) representing the actual physiological state of the patient, at least one target parameter defined by the user, and/or pre-defined data set by the manufacturer, and/or the step of processing the data to a control signal for said actuator with a processing unit which reads the data of said memory and/or the provided data.
43. The method according to any of the aspects 38 to 42, wherein the at least one additional parameter (AP) comprises
   an absorbing capacity (AC) representing the patient's capacity to absorb additional infusion fluid,
   a reduction rate (RR) representing the patient's capacity to reduce the infusion fluid in the body, and/or
   a patient-state-index (PSI).
44. The method according to any of the aspects 38 to 43, wherein at least three additional parameter (AP) represent the actual physiological state of the patient and wherein three additional parameters are: absorbing capacity (AC), reduction rate (RR) and the patient-state-index (PSI).
45. The method according to any of the aspects 38 to 44, wherein at least three additional parameter (AP) represent the actual physiological state of the patient and wherein three additional parameters are: amount of extravascular lungwater, central venous pressure and change of heart rate.
46. The method according to any of the aspects 38 to 45, wherein the at least one additional parameter (AP) or the absorbing capacity (AC) comprises at least one or several of the parameters: amount of extravascular lungwater, central venous pressure, intracranial pressure, intraabdominal pressure, compartment pressure, heart rate, heart rate variability, blood pressure, arrhythmia, proBNP-level, ejection fraction of heart, ultrasonic filling status of heart and/or a capillary leak-index representing the value of a capillary leak syndrome.
47. The method according to any of the aspects 38 to 46, wherein the at least one additional parameter (AP) or the reduction rate (RR) comprises at least one or several of the parameters: glomerular filtration rate, creatinin clearance, creatinin level, urea clearance, urea level, elimination, and/or elimination rate.
48. The method according to any of the aspects 38 to 47, wherein the at least one additional parameter (AP) or the patient-state-index (PSI) comprises at least one or several of the patient-state-index parameter(s) (PSIP), the patient-state-index parameter(s) (PSIP) comprising the parameters: peripheral perfusion, splachnik perfusion, glomerular filtration rate, creatinin clearance, creatinin level, creatinin level change rate, urea clearance, urea level, urea level change rate, intracranial pressure, change-rate of intracranial pressure, change-rate of intraabdominal pressure, intraabdominal pressure, change-rate of compartment pressure, compartment pressure, heart rate, heart rate variability, arrhythmia, BNP level, BNP level change rate, NTproBNP-level, NTproBNP level change rate, ejection fraction of heart, ultrasonic filling status of heart, elimination, elimination change rate.
49. The method according to any of the aspects 38 to 48, wherein said data processing unit (12) calculates the patient-state-index (PSI), the calculation comprises the assessment, prioritization, and/or weighting of at least one or several or all patient-state-index parameters (PSIP).
50. The method according to any of the aspects 38 to 49, wherein said data processing unit (12) calculates at least one additional parameter representing the actual physiological state of the patient, said calculation comprises the assessment, prioritization, and/or weighting of at least two of the parameters: absorbing capacity (AC), reduction rate (RR) and/or patient-state-index (PSI) or parameters thereof.
51. The method according to any of the aspects 38 to 50, wherein said data processing unit (12) calculates at least one additional parameter representing the actual physiological state of the patient, said calculation comprises the assessment, prioritization, and/or weighting of at least two of the parameters: change of heart rate, central venous pressure and/or amount of extravascular lungwater.
52. The method according to any of the aspects 38 to 51, wherein said data processing unit (12) is adapted to prioritize three additional parameters (AP) representing the actual physiological state of the patient, wherein the additional parameters (AP) amount of extravascular lungwater, central venous pressure and change of heart rate are prioritized, and wherein the infusion is interrupted when at least one of said additional parameter (AP) is above a threshold value.
53. The method according to any of the aspects 38 to 52, wherein additional parameters (AP) of second priority are part of the patient-state-index (PSI), and wherein the infusion is interrupted when patient-state-index (PSI) is above a threshold value.
54. The method according to any of the aspects 38 to 53, wherein the user selects and/or prioritize the parameters being an at least one additional parameter (AP) representing the actual physiological state of the patient and/or being part of the patient-state-index (PSI).
55. The method according to any of the aspects 38 to 54, wherein said control unit (10) controls via said actuator (13) at least one infusion parameter, preferably the flow rate (FR) and/or the temperature of the infusion fluid, in accordance with the target body temperature (Tb, target), preferably a preset target body temperature (Tb, target, preset), and/or a target body temperature profile.
56. The method according to any of aspects 38 to 55, wherein the target body temperature profile is a user defined profile, preferably based on at least a preset target body temperature (Tb, target, preset), a change rate of body temperature (Tb), and/or target flow rate (FR)
57. The method according to any of the aspects 38 to 56, wherein said control unit (10) controls via said actuator (13) the infusion parameters within at least one upper and/or lower threshold value of the infusion parameters, and/or
   controls via said actuator (13) the actual body temperature (Tb) and/or the change rate of the actual body temperature (Tb) within at least one upper and/or lower threshold value.
58. The method according to any of the aspects 38 to 57, wherein said control unit (10) controls via said actuator (13) the at least one infusion parameter in accordance with at least one upper and/or lower threshold value of the at least one additional parameter (AP), preferably the absorbing capacity (AC), reduction rate (RR) and/or patient-state-index (PSI) or parameters thereof, most preferably upper and/or lower threshold values of the change of heart rate, central venous pressure and/or amount of extravascular lungwater.
59. The method according to any of the aspects 38 to 58, wherein no fluid is infused to the patient in the event that at least one additional parameter (AP), at least one infusion parameter, the actual body temperature (Tb) and/or the change rate of the actual body temperature (Tb) is/are not within the respective upper and/or lower threshold value(s).
60. The method according to any of the aspects 38 to 59, wherein at least one target value and/or at least one threshold value for the at least one additional parameter (AP), for the at least one infusion parameter, actual body temperature (Tb), and/or the change rate of the actual body temperature (Tb) is/are administered by the user and/or is/are pre-defined data set by the manufacturer.
61. The method according to any of the aspects 38 to 60, wherein said control unit (10) adapts the target body temperature (Tb) and/or the target temperature profile only when the actual target body temperature (Tb) and/or the target temperature profile cannot be achieved by a setting of the at least one infusion parameter, which is within the upper and/or lower threshold value(s) for the at least one infusion parameter, and which ensures that the at least one additional parameter and/or the temperature change rate are within the respective upper and/or lower threshold value(s), and/or
   when the change rate of the actual body temperature (Tb) is not within its upper and/or lower threshold value.
62. The method according to any of the aspects 38 to 61, wherein, in the event of a target body temperature below the actual body temperature (Tb), said control unit (10) controls said actuator (13) so as
   to reduce the flow rate (FR) and to reduce the temperature of the infusion fluid thereby keeping the actual body temperature (Tb) at the target body temperature when the at least one additional parameter (AP), preferably the reduction rate (RR), the absorbing capacity (AC) and/or a patient-state-index (PSI), and most preferably the change of heart rate, central venous pressure and/or amount of extravascular lungwater, is/are above a first upper threshold value,
   and/or
   increase the flow rate (FR) and to increase the temperature of the infusion fluid thereby keeping the actual body temperature (Tb) at the target body temperature when the at least one additional parameter (AP), preferably the reduction rate (RR), the absorbing capacity (AC) and/or a patient-state-index (PSI), and most preferably the change of heart rate, central venous pressure and/or amount of extravascular lungwater, is/are below a first lower threshold value.
63. The method according to any of the aspects 38 to 62, wherein, in the event of a target body temperature below the actual body temperature (Tb), said control unit (10) controls said actuator (13) so as
   to reduce the flow rate (FR) and to reduce the temperature of the infusion fluid thereby keeping the actual body temperature (Tb) at a reduced target body temperature when the at least one additional parameter (AP), preferably the reduction rate (RR), the absorbing capacity (AC) and/or a patient-state-index (PSI), and most preferably the change of heart rate, central venous pressure and/or amount of extravascular lungwater, is/are above a second upper threshold value.
64. The method according to any of the aspects 38 to 63, wherein, in the event of a target body temperature below the actual body temperature (Tb), said control unit (10) stops the supply of infusion to the patient in the event of at least one additional parameter (AP), preferably the reduction rate (RR), the absorbing capacity (AC) and/or a patient-state-index (PSI), and most preferably the change of heart rate, central venous pressure and/or amount of extravascular lungwater, below a third upper threshold value.
65. The method according to any of the aspects 38 to 64, wherein the device (1) triggers an alarm and/or to display an information and/or different status information when the at least one lower and/or upper threshold value is achieved.
66. The method according to any of the aspects 38 to 65, wherein
   the first lower threshold value is a central venous pressure of 12 cm H2O,
   the second lower threshold value is a central venous pressure of 20 cm H2O,
   and the third lower threshold value is a central venous pressure of 35 cm H2O.
67. The method according to any of the aspects 38 to 66, wherein the third upper threshhold value is a change of heart rate of 20 beats per minute with a heart beat in the range of 60 to 180 beats per minute.
68. The method according to any of the aspects 38 to 67, wherein the device (1) triggers an alarm and/or to display an information and/or different status information when the amount of infusion left in said supply (1) is below a minimum value, and/or the remaining period of time until the infusion needs to be refilled is below a minimum value.
69. The method according to any of the aspects 38 to 68, wherein the fluid temperature input (3), the body temperature input (5), additional input (7), and/or the flow rate input (9) comprise(s):
   at least one sensor element (4), and/or
   at least one connector (6) for connecting at least one sensor element (4) and/or at least one medical device (8), wherein
   the at least one sensor element (4) and/or at least one connector (6) receive, measure, and/or process the actual temperature of the infusion fluid (Tfa), the actual body temperature of the patient (Tb), the at least one additional parameter representing the actual physiological state of the patient (AP) and/or the actual flow rate (FR).

The present invention will become more fully understood from the detailed description given hereinafter and the accompanying drawings which are given by way of illustration only, and thus, are not limitative of the present invention, and wherein:
- Figure 1: shows a schematic view of one embodiment of the present invention,
- Figure 2: shows a schematic view of another embodiment of the present invention,
- Figure 3: shows a profile of the target body temperature,
- Figure 4: is a flow chart showing some of the steps carried out by the control unit,
- Figure 5: is an schematic overview of the additional parameters,
- Figure 6: shows exemplarily the relation between some infusion parameters and some additional parameters, respectively,
- Figure 7: shows a temperature profile of the target body temperature Tb, target which was revised at a given time ta.

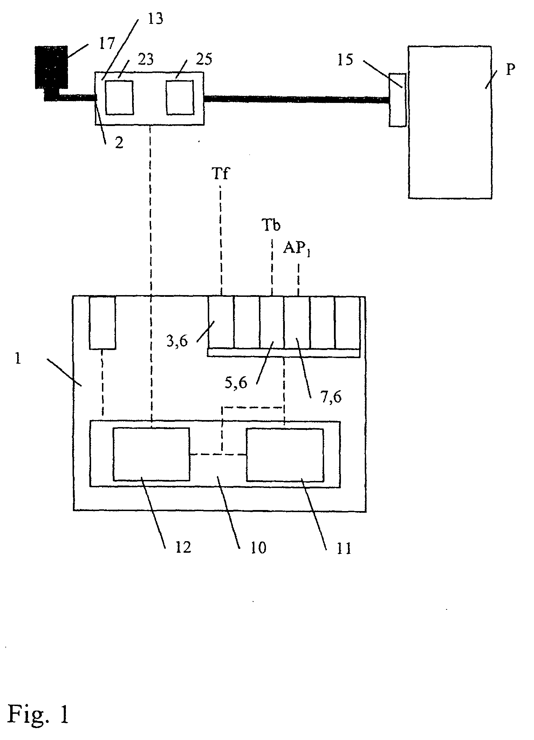

Figure 1 shows a device 1 for controlling a temperature of a patient by an infusion of fluid.

The device 1 comprises a control unit 10 with a memory 11 and a processing unit 12. The processing unit 12 communicates with memory I and connectors 6.

In this embodiment, the device comprises seven connectors out of which one is used as a fluid temperature input 3, another one is used as the body temperature input 5 and a third one is used as an additional input 7 which is adapted to receive at least one additional parameter AP representing the actual physiological state of the patient. The connectors 6 could be any kind of standard connector used for connecting any kind of cabling to a device. Alternatively, the connector may be replaced by any suitable wireless data transfer means.

In this embodiment the fluid temperature input 3 receives the actual temperature Tfa of the infusion fluid which may be provided by any suitable sensing means. Body temperature input 5 and additional input 7 receive the actual body temperature Tb and the at least one additional parameter AP representing the actual physiological state of the patient, respectively, by any suitable means and as exemplified before.

The control unit 10 is moreover communicating with an actuator 13 which comprises a temperature actuator 23 and a flow rate actuator 25. The temperature actuator 23 is embodied as a heat exchanger which adjusts the temperature of the infusion fluid. Flow rate actuator 25 is embodied as a pump which adjusts the flow rate of the infusion fluid to be infused.

Device 1 and here actuator 13 has a supply 2 configured as an inlet which is connected via a tube with a reservoir 17 being embodied as an infusion bag. Actuator 13 is moreover connected via a tube to a patient connector 15 which may be an infusion needle as commonly known by the art. Actuator 13 is configured in some embodiments to have or to surround a tube which connects reservoir 17 to connector 15 and the actuators are acting on the infusion fluid from the outside, for instance via a hose pump. The cleaning, sterilization and/or replacement is then advantageously facilitated and the high hygienic requirement are met. In other embodiments two separate tubes are used and the actuators are in direct contact with the infusion fluid.

The control unit 10 controls actuator 13 based on the target body temperature Tb, target the actual temperature of the infusion fluid, the actual body temperature Tb, the actual flow rate FR and one additional parameter AP1. The additional parameter AP1 could be any parameter representing the physiological state of the patient. In this embodiment the one additional parameter is a parameter which represents the absorption capacity of the patient, namely the central-venous pressure. The infusion fluid, thus flows from the infusion bag 17 into actuator 13 in which the temperature and/or the flow rate of the fluid may be adjusted. The infusion fluidmay be infused via infusion needle 15.

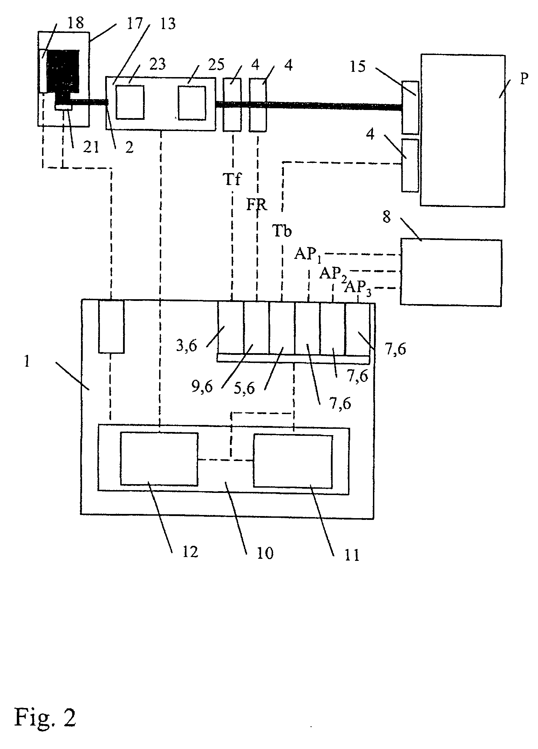

Figure 2 shows an embodiment with some modifications compared to the embodiment of Figure 1. Hereafter mainly the differences to the embodiment of Figure 1 are highlighted. The reference numerals of those parts of Figure 2 which are identical to those of Figure 1 are identical.

In Figure 2 fluid temperature input 3, body temperature input 5 and flow rate input 9 are in direct communication with suitable sensors 4, respectively. Moreover, three additional inputs 7 are connected to a medical device 8 which provides three additional parameter AP1, AP2, AP3 representing the actual physiological state of the patient. In the present embodiment, the three additional parameters AP1, AP2, AP3 are the amount of extra-vascular lung water, the central-venous pressure and the change of heart rate.

The data received by the device 1 are used by control unit 10 to generate a control signal for actuator 13 which adjusts the temperature and/or the flow rate of the infusion fluid to be infused. The embodiment according to Figure 2 moreover comprises a temperature regulating means 18 such as a heating and/or cooling element which is part of reservoir 17 here embodied as a thermobox.

A conventional infusion bag is positioned in thermobox 17 and connected to a tube which is in communication with inlet 2. Moreover, the thermobox 17 comprises a supply temperature sensor 21 which provides the temperature of the infusion fluid stored in the thermobox 17. The supply temperature sensor 21 and the temperature regulating device 18 are in electronic communication with the control unit 10. In this embodiment, the control unit 10 not only adjusts the temperature and flow rate of the fluid to be infused by actuator 13 but also controls the temperature of the infusion fluid stored in the infusion bag located in thermobox 17.

The temperature of the infusion fluid in the infusion bag is generally regulated to a value which is generally suitable for the infusion and temperature actuator 23 preferably only conducts a final adjustment to the desired temperature of the fluid to be infused. However, it may also be possible to configure the temperature actuator 23 and the temperature regulating device 18 so that the workload is equally shared among them.

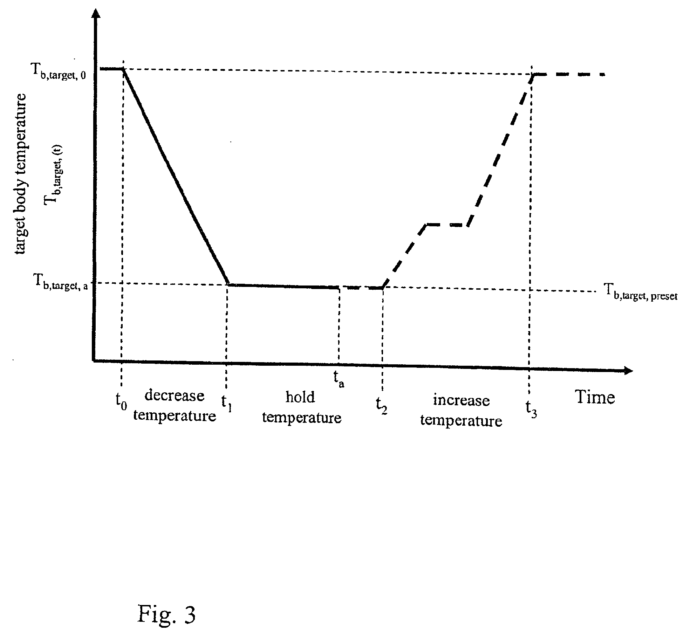

Figure 3 shows a temperature profile of the target body temperature Tb, target (t). The temperature control generally starts with a initial target body temperature Tb, target, 0 which is equal to the actual body temperature Tb of the patient. The target body temperature then decreases with a preset change rate of body temperature until a lower preset target body temperature Tb, target, preset is reached at a time t1. The preset target body temperature Tb, target, preset will be constant until a given time t2. Afterwards, the target body temperature Tb, target is herein increased in two steps. The treatment is finished at a time t3 in which the target body temperature Tb, target is equal to the body temperature of the patient without temperature control.

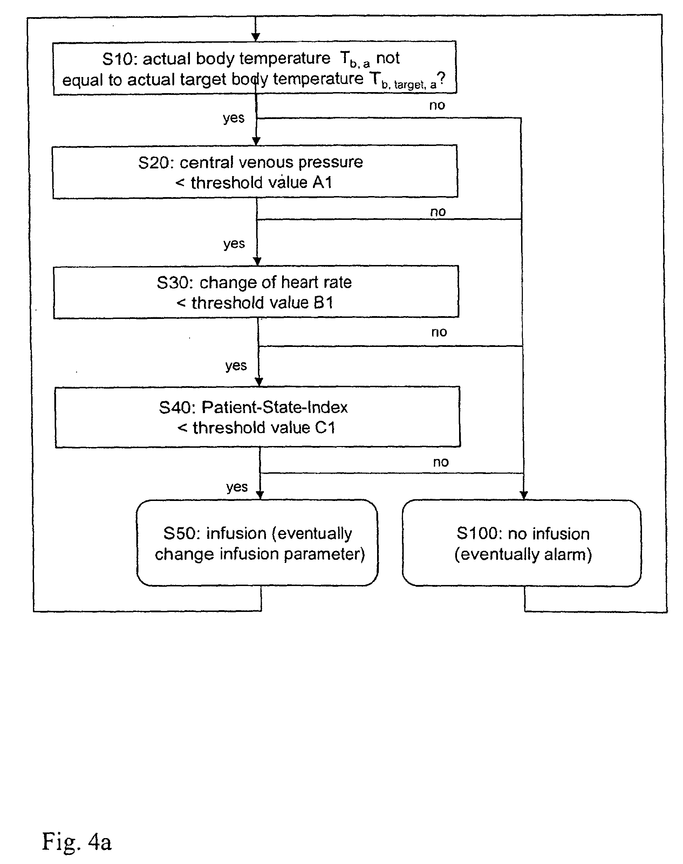

Figure 4a depicts some of the steps carried out by control unit 10 in order to control the temperature of a patient by an infusion of a fluid. In step S 10, the actual body temperature Tb, a in a given point ta as, for instance, shown in Figure 3 is compared to an actual target body temperature Tb, target, a. If the actual body temperature Tb, a is not equal to the actual target body temperature Tb, target, a then the central venous pressure is compared to a threshold value A of the central-venous pressure in a step S20. If the central venous pressure is lower than the respective threshold value A then the change of heart rate is compared to a threshold value B of the change of heart rate in a step S30. If the change of heart rate is lower than a threshold value B then a patient-state-index is compared to a threshold value C of the patient state index in step S40. If the patient-state-index is lower that the respective threshold value C then the infusion will be carried out in step S50. Eventually, one or several infusion parameters, here the temperature of the fluid to be infused and/or the flow rate FR are adjusted in step S50 and the control unit 10 restarts the routine with S10.

If in the steps S20, S30, S40 any of the values for the central venous pressure, change of heart rate, and/or patient-state-index is above the respective threshold value A1, B1 and/or C1 no infusion fluid will be infused in step S 100 and, eventually, the device will trigger an alarm.

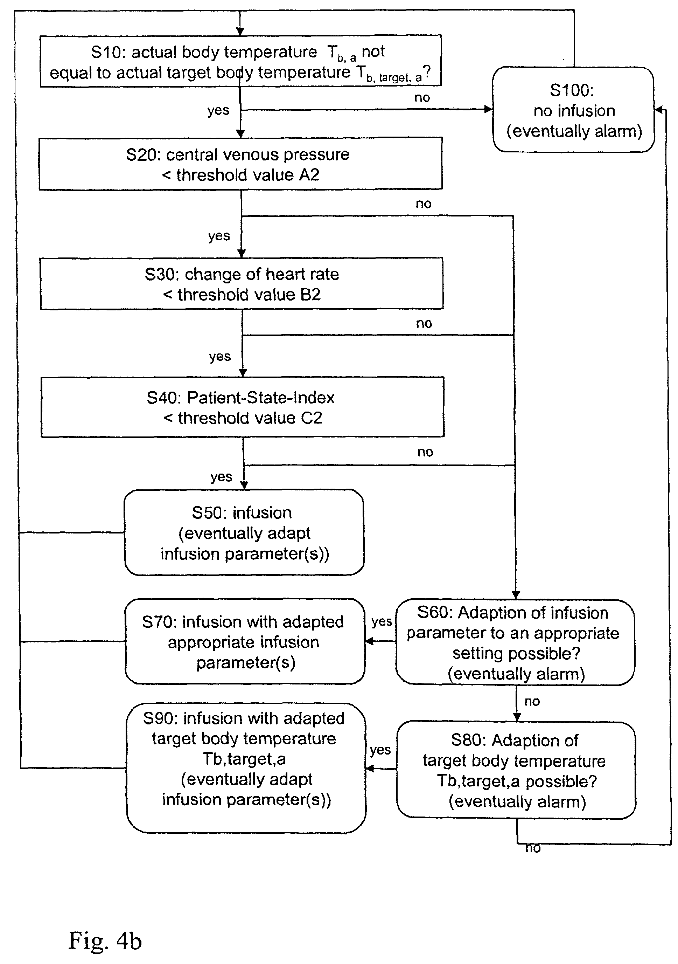

Figure 4b also depicts some of the steps carried out by control unit 10 in order to control the temperature of a patient by an infusion of a fluid. The steps S10, S20, S30, S40, S50 are identical to those of the embodiment in accordance to Figure 4a.

If in the steps S20, S30, S40 any of the values for the central venous pressure, change of heart rate, and/or patient-state-index is above the respective threshold value A2, B2 and/or C2 the control unit 10 does not immediately stop the infusion fluid but evaluates in step S60 whether the infusion parameter(s) may be adapted to an appropriate setting, which further allows the infusion of fluid within the threshold parameter A2, B2, C2 and eventually within the threshold value(s) for the infusion parameter(s). The flow rate FR may, for instance, be decreased to reduce the central venous pressure and the temperature of the fluid may be further decreased at the same time in order to obtain the same body temperature regulating effect in the event of cooling a patient. If the infusion with an adapted appropriate infusion parameter setting is possible, then the infusion continues with an adapted appropriate infusion parameter setting in step S70 and the control unit 10 restarts the routine with S10.

If the infusion with an adapted appropriate infusion parameter setting is not possible, then the control unit 10 evaluates in step S80 whether the infusion may be possible with an adapted target body temperature Tb, target, a, eventually together with an adapted appropriate infusion parameter setting. For instance, in the event of cooling a patient, the required flow rate FR may be further reduced if the adapted target body temperature Tb, target, a is closer to the normal body temperature of the uncooled patient which would additionally decrease the central venous pressure. If the infusion is possible with an adapted target body temperature Tb, target, a, eventually together with an adapted appropriate infusion parameter setting then the infusion continues with an adapted target body temperature Tb, target, a, eventually together with an adapted appropriate infusion parameter setting in step S90 and the control unit 10 restarts the routine with S10. If an infusion with an adapted target body temperature Tb, target, a, eventually together with an adapted appropriate infusion parameter setting, is not possible then no fluid is infused in step S100). The control unit moreover eventually triggers an alarm during step S60, S80, and/or S100.

The embodiments of Figure 4a and Figure 4b could also be combined. For instance with threshold values A2, B2, C2 which are lower than threshold values A1, B1, C1. In such an embodiment, the control unit 10 may first evaluate an appropriate infusion parameter setting and/or an adapted target body temperature Tb, target, a, if any one of the threshold values A2, B2, C2 is reached and may only stop the infusion, if no appropriate infusion parameter setting and no adapted target body temperature Tb, target, a is possible or the threshold values A1, B1, C1 are reached, provided that the actual body temperature Tb, a is not equal to the actual target body temperature Tb, target, a.

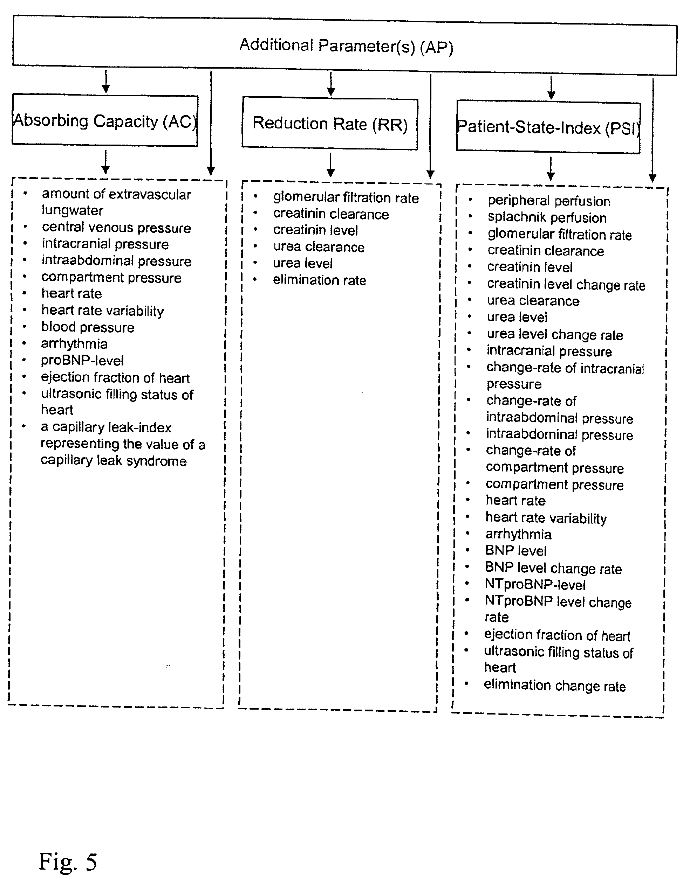

Figure 5 shows exemplarily some of the additional parameters which represents the actual physiological state of the patient. The additional parameters may be subdivided into three groups. The first group concerning the absorbing capacity AC represents the patient's ability to absorb infusion fluid. The second group is the reduction rate RR which represents parameters indicative for the patient's ability to reduce fluid contained in the body. A third group of parameters are indicative for the general condition of the patient which are in the present embodiment associated to a patient-state-index.

The patient-state-index is here an index which considers several parameters for the calculation of the patient-state-index value. The patient-state-index value has here the same priority as the reduction rate RR and/or the absorbing capacity AC, for instance exemplified by the central-venous pressure and/or the change of heart rate. It is also possible that the control unit 10 only considers one or several additional parameters AP without calculating a patient-state-index PSI. The controller might, for instance, evaluate in a step S40 of Figure 4a or 4b not the patient-state-index but the amount of extra-vascular lung water. Also, the amount of extra-vascular lung water could be evaluated in an additional step S45.

The vertical arrows in Figure 5 symbolize that each parameter listed within the respective rectangles with a broken line could be an additional parameter AP. Moreover each parameter listed in the left rectangle with a broken line could represent an absorbing capacity AC, each parameter listed in the center rectangle with a broken line could represent an reduction rate RR, and each parameter listed in the right rectangle with a broken line could represent a parameter for the calculation of the patient-state-index.

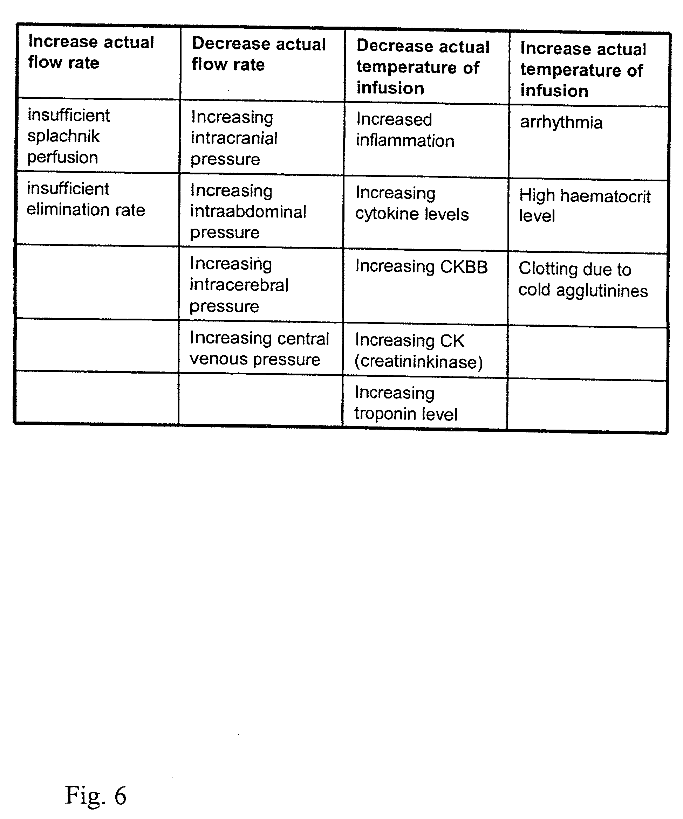

Figure 6 shows some of the additional parameters AP and their impact on the infusion parameters flow rate FR and/or temperature of infusion fluid.

The splanchnic perfusion may be an indicator for the actual volume status and/or peripheral resistance. Knowing the volume status and/or the peripheral resistance is vital for controlling the body temperature by infusion. If the splanchnic perfusion is insufficient the flow rate FR should preferably be increased.

The elimination rate indicates how much physiological waste can be eliminated in a specific period of time. It also partly indicates the metabolism of the body. An insufficient elimination rate would require the decrease the actual flow rate.

The intra-cranial pressure is dependent on several factors such as the heart capacity, the volume status, the blood pressure, edema etc. The intra-cranial pressure is influenced by the infusion of fluid and should not be increased above a certain value. Thus, the actual flow rate FR should be decreased if the intra-cranial pressure is increased or increasing.

Similar to intra-cranial pressure, the intra-cerebral pressure depends on several factors such as heart capacity, volume status, blood pressure, edema, etc. Thus, it is important not to increase the intra-cerebral pressure above a certain limit. Consequently, the actual flow rate FR of the fluid should be decreased when the intra-cerebral pressure is increased or increasing.

The intra-abdominal pressure may be an indication for the volume status of a patient especially in cases of ascites. It is therefore important not to increase the intra-abdominal pressure to much by an infusion in order to avoid complications like the compartment syndrome. Therefore, the actual flow rate FR of the infused fluid should be decreased in the case of an increased or increasing intra-abdominal pressure.

The central-venous pressure is an indicator for the volume status of the body and the volume capacity of the heart. A increased or increasing central-venous pressure indicates a limited capacity of the body for further absorption of infusion fluid. Thus, the actual flow rate FR of the infusion fluid to be infused into the patient should be reduced when a central-venous pressure is increased or increasing.

Troponin is an indicator for heart cell death. In order to avoid heart cell death, the actual flow rate should be decreased in the case of an increasing troponin level. Alternatively or additionally, the actual temperature of the infusion may be decreased in the case of an increased or increasing troponin level.

Inflammatory processes may be influenced by the temperature of the infused fluid. By lowering the temperature, it is possible to significantly decelerate the inflammation and prevent inflammatory primary and/or secondary damages. Thus, the actual temperature of the infused fluid may be decreased in the case of an increased or increasing inflammation and vice-versa.

Cytokine levels are inter alia an indication for inflammation, cell death, infection, sepsis, etc. By lowering the temperature it is possible to significantly slower the metabolism and the results of cytokines, and cytokine production. Thus, the actual temperature of the infusion fluid may be decreased in the case of an increased or increasing cytokine levels.

Arhythmia may be an indicator for disturbed signal transmission due to a low temperature of the body. Since it is important not to disturb the signal transmission the actual temperature of the infusion should be increased when arrhythmia occurs.

Lowering the temperature the body leads to a higher viscosity which may lead to clotting. Since it is important to keep the blood fluidly enough the actual temperature of the infused fluid may be increased in the case of an increased or increasing haematocrite level.

Some patients may have cold agglutinines which would lead to intra-vasal clotting. In order to avoid intra-vasal clotting the actual temperature of the infusion may be increased in the case of clotting due to cold agglutinines.

The functional relationship between the infusion parameter(s) and aforementioned parameters such as, for instance, the central venous pressure are described herein only in one direction, for instance, that the flow rate should be decreased in the case of an increasing or increased central venous pressure. It is readily understood, that the same functional relationship applies inversely, for instance that the flow rate could be increased in the case of a low or non critical central venous pressure.

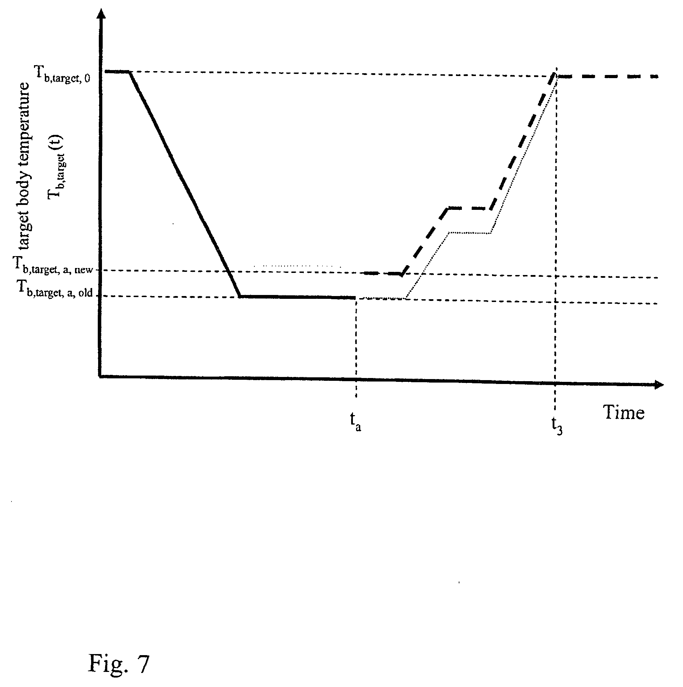

Figure 7 shows a target body temperature Tb, target (t) as a function of time. The solid bold line representing the target body temperature Tb, target (t) until a point of time ta and a dotted line representing the target body temperature Tb, target (t) until a point of time t3 correspond to the target body temperature profile of Figure 3. The control unit 10 could not change the infusion parameter(s) at the point of time ta to an appropriate setting so that the infusion parameter(s) and/or the additional parameter(s) AP are within the respective threshold value (s). For instance, the control unit 10 could not find a setting in step S60 of Figure 4b which ensures that a central venous pressure is below a threshold value A2 as evaluated in step S20 of Figure 4b and/or a change of heart rate being below a threshold value B2 as evaluated in step S30 of Figure 4b and/or a patient-state-index PSI which is below a threshold value C2 as evaluated in step S40 of Figure 4b. In other words, instead of stopping the infusion as done in step S 100 of Figure 4a, the control unit 10 calculates a new target body temperature Tb, target, a, new and thus a new temperature profile which is depicted as a bold broken line in Figure 7. The target body temperature Tb, target, a, new as well as the new target body temperature profile are calculated by the control unit 10 so that the threshold values of the infusion parameter(s) and/or of the additional parameter(s) AP will presumably not be reached. Additionally, the device will inform the user about the new target body temperature profile.

In the claims, the term "comprises/comprising" does not exclude the presence of other elements or steps.

## Claims

1. A device (1) for controlling or assisting to control a temperature of a patient by infusion of fluid, comprising
at least one supply (2) of infusion fluid;
at least one body temperature input (5) adapted to receive the actual body temperature (Tb) of the patient;
at least one additional input (7) adapted to receive at least one additional parameter (AP) representing the actual physiological state of the patient;
at least one control unit (10) communicating with said body temperature input (5), and said additional input (7); and
at least one actuator (13) which controls at least the actual flow rate (FR) and actual temperature of the infusion fluid in accordance with at least one control signal of said control unit (10);
wherein said control unit (10) controls the temperature of a patient with said actuator (13) based on
- at least a target body temperature (Tb,target),
- the actual temperature (Tfa) and/or the defined temperature (Tfd) of the infusion fluid,
- the actual body temperature (Tb),
- the actual flow rate (FR), and
- the at least one additional parameter (AP) representing the actual physiological state of the patient'; **characterised in that**
the at least one additional parameters (AP) includes at least the parameter change of heart rate;
wherein, in the event of a target body temperature below the actual body temperature (Tb), said control unit (10) is adapted to control said actuator (13) so as to reduce the flow rate (FR) and to reduce the temperature of the infusion fluid thereby keeping the actual body temperature (Tb) at the target body temperature when the at least one additional parameter (AP) is above a first upper threshold value,
and/or
to increase the flow rate (FR) and to increase the temperature of the infusion fluid thereby keeping the actual body temperature (Tb) at the target body temperature when the at least one additional parameter (AP) is below a first lower threshold value.

2. The device (1) according to claim 1, wherein the device (1) comprises at least one fluid temperature input (3) communicating with said control unit (10) and adapted to receive the actual temperature (Tfa) of the infusion fluid, and/or a defined value for the temperature (Tfd) of the infusion fluid.

3. The device (1) according to any of the preceding claims, wherein the device (1) comprises at least one flow rate input (9) communicating with said control unit (10) and adapted to receive the actual flow rate (FR) of the infusion fluid, and/or wherein the device (1) derives the actual flow rate (FR) of the infusion fluid based on the settings of actuator (13).

4. The device (1) according to any of the preceding claims, wherein said control unit (10) comprises:
a memory (11) adapted to store data, and/or
a data processing unit (12) adapted to read the data of said memory (11) and/or of said inputs (3, 5, 7, 9) and process the data to a control signal for said actuator (13).

5. The device (1) according to any of the preceding claims, wherein the at least one additional parameter (AP) comprises
an absorbing capacity (AC) representing the patient's capacity to absorb additional infusion fluid,
a reduction rate (RR) representing the patient's capacity to reduce the infusion fluid in the body, and/or
a patient-state-index (PSI).

6. The device (1) according to any of the preceding claims, wherein at least three additional parameters (AP) represent the actual physiological state of the patient, and wherein the three additional parameters are: amount of extravascular lungwater, central venous pressure and change of heart rate.

7. The device (1) according to claim 5, wherein the absorbing capacity (AC) is at least one or several of the parameters: amount of extravascular lungwater, central venous pressure, intracranial pressure, intraabdominal pressure, compartment pressure, heart rate, heart rate variability, blood pressure, arrhythmia, proBNP-level, ejection fraction of heart, ultrasonic filling status of heart and a capillary leak-index representing the value of a capillary leak syndrome.

8. The device (1) according to claim 5, wherein the reduction rate (RR) is at least one or several of the parameters: glomerular filtration rate, creatinin clearance, creatinin level, urea clearance, urea level, elimination of physiological waste, and elimination rate.

9. The device (1) according to any of the preceding claims, wherein the at least one additional parameter (AP) comprises at least one or several of the parameters:
amount of extravascular lungwater, central venous pressure, blood pressure, capillary leak-index representing the value of a capillary leak syndrome, elimination rate,
peripheral perfusion, splachnik perfusion, glomerular filtration rate, creatinin clearance, creatinin level, creatinin level change rate, urea clearance, urea level, urea level change rate, intracranial pressure, change-rate of intracranial pressure, change-rate of intraabdominal pressure, intraabdominal pressure, change-rate of compartment pressure, compartment pressure, heart rate, heart rate variability, arrhythmia, BNP level, BNP level change rate, proBNP-level, NTproBNP-level, NTproBNP level change rate, ejection fraction of heart, ultrasonic filling status of heart, elimination of physiological waste, and elimination change rate.

10. The device (1) according to any of the preceding claims 5 to 9, wherein said data processing unit (12) is adapted to calculate the patient-state-index (PSI), the calculation comprises the assessment, prioritization and/or weighting of at least two or several or all patient-state-index parameters (PSIP), wherein the patient-state-index (PSI) is calculated with at least one or several of the patient-state-index parameter(s) (PSIP): peripheral perfusion, splachnik perfusion, glomerular filtration rate, creatinin clearance, creatinin level, creatinin level change rate, urea clearance, urea level, urea level change rate, intracranial pressure, change-rate of intracranial pressure, change-rate of intraabdominal pressure, intraabdominal pressure, change-rate of compartment pressure, compartment pressure, heart rate, heart rate variability, arrhythmia, BNP level, BNP level change rate, NTproBNP-level, NTproBNP level change rate, ejection fraction of heart, ultrasonic filling status of heart, elimination of physiological waste and elimination change rate.

11. The device (1) according to any of the preceding claims 5 to 10, wherein said data processing unit (12) is adapted to calculate at least one additional parameter (AP) representing the actual physiological state of the patient, said calculation comprises the assessment, prioritization and/or weighting of at least two of the parameters of absorbing capacity (AC), reduction rate (RR) and/or patient-state-index (PSI).

12. The device (1) according to any of the preceding claims 5 to 11, wherein said data processing unit (12) is adapted to calculate at least one additional parameter (AP) representing the actual physiological state of the patient, said calculation comprises the assessment, prioritization and/or weighting of at least two of the parameters: change of heart rate, central venous pressure and/or amount of extravascular lungwater.

13. The device (1) according to any of the preceding claims 5 to 12, wherein said data processing unit (12) is adapted to prioritize three additional parameters (AP) representing the actual physiological state of the patient, wherein the additional parameters (AP) amount of extravascular lungwater, central venous pressure and change of heart rate are prioritized, and wherein the infusion is interrupted when at least one of said additional parameter (AP) is above a threshold value.

14. The device (1) according to any of the preceding claims 6 to 13, wherein additional parameters (AP) of second priority are part of the patient-state-index (PSI), and wherein the infusion is interrupted when the patient-state-index (PSI) is above a threshold value.

15. The device (1) according to any of the preceding claims, wherein the user prioritize the parameters being an at least one additional parameter (AP) representing the actual physiological state of the patient and/or being part of the patient-state-index (PSI).

16. The device (1) according to any of the preceding claims 2 to 15, wherein sai control unit (10) controls via said actuator (13) at least two infusion parameters, including the flow rate (FR) and the temperature of the infusion fluid, in accordance with the target body temperature (Tb,target), preferably a preset target body temperature (Tb, target, preset) and/or a target body temperature profile.

17. The device (1) according to claim 16, wherein the target body temperature profile is a user defined profile, preferably based on at least a preset target body temperature (Tb,target, preset), a change rate of body temperature (Tb), and/or target flow rate (FR)

18. The device (1) according to any of the preceding claims, wherein said control unit (10) controls via said actuator (13) the at least two infusion parameters within at least one upper and/or lower threshold value of the at least two infusion parameters, and/or controls via said actuator (13) the actual body temperature (Tb) and/or the change rate of the actual body temperature (Tb) within at least one upper and/or lower threshold value.

19. The device (1) according to any of the preceding claims 16 to 18, wherein said control unit (10) controls via said actuator (13) the at least two infusion parameters in accordance with at least one upper and/or lower threshold value of the at least one additional parameter (AP).

20. The device (1) according to any of the preceding claims 16 to 19, wherein said control unit (10) adapts the target body temperature (Tb,target) and/or the target body temperature profile
when the actual target body temperature (Tb,target) and/or the target body temperature profile cannot be achieved by a setting of the at least one infusion parameter, which is within the upper and/or lower threshold value(s) for the at least two infusion parameters, and which ensures that the at least one additional parameter and/or the temperature change rate are within the respective upper and/or lower threshold value(s), and/or when the change rate of the actual body temperature (Tb) is not within its upper and/or lower threshold value.

21. The device (1) according to any of the preceding claims, wherein at least one target value and/or at least one threshold value for the at least one additional parameter (AP), for the at least two infusion parameters, for the actual body temperature (Tb), and/or for the change rate of the actual body temperature (Tb) is/are administered by the user and/or is/are pre-defined data set by the manufacturer.

22. The device (1) according to any of the preceding claims, wherein the device (1) is adapted to trigger an alarm and/or to display an information or different status information when at least one lower or upper threshold value is achieved.

23. The device (1) according to any of the preceding claims, wherein the fluid temperature input (3), the body temperature input (5), additional input (7), and/or the flow rate input (9) comprise(s):
at least one sensor element (4), and/or
at least one connector (6) for connecting at least one sensor element (4) and/or at least one medical device (8), wherein
the at least one sensor element (4) and/or at least one connector (6) is/are adapted to receive, measure, and/or process the actual temperature (Tfa) of the infusion fluid, the actual body temperature of the patient (Tb), the at least one additional parameter (AP) representing the actual physiological state of the patient, the actual flow rate (FR) and/or any other parameter.

24. The device (1) according to any of the preceding claims, wherein said at least one actuator (13) is in fluid communication with said supply (2), and/or wherein said supply (2) is connectable and/or comprises at least one reservoir (17), wherein said reservoir (17) is preferably an infusion bag.

25. The device (1) according to claim 24, wherein said reservoir (17) is connectable and/or comprises at least one temperature regulating device (18) and/or at least one supply temperature sensor (21).

26. The device (1) according to claim 25, wherein said temperature regulating device (18) and/or said supply temperature sensor (21) is/are in communication with said control unit (10).

27. The device (1) according to any of the preceding claims, wherein the defined temperature (Tfd) of the infusion fluid is entered by a user or obtained by an external device, preferably obtained by said temperature regulating device (18) and/or said supply temperature sensor (21).

28. The device (1) according to any of the preceding claims, wherein said device (1) comprises and/or is connectable to at least one patient connector (15) which connects said actuator (13) with the patient.

## Patentansprüche

1. Vorrichtung (1) zur Steuerung oder Unterstützung der Steuerung einer Temperatur eines Patienten durch Infusion von Fluid, die aufweist:
mindestens eine Zufuhr (2) von Infusionsfluid;
mindestens einen Körpertemperatureingang (5), der geeignet ist, die Ist-Körpertemperatur (Tb) des Patienten zu empfangen;
mindestens einen Zusatzeingang (7), der geeignet ist, mindestens einen den physiologischen Ist-Zustand des Patienten repräsentierenden Zusatzparameter (AP) zu empfangen;
mindestens eine Steuereinheit (10), die mit dem Körpertemperatureingang (5) und dem Zusatzeingang (7) kommuniziert; und
mindestens einen Aktuator (13), der mindestens den Ist-Durchfluss (FR) und die Ist-Temperatur des Infusionsfluids in Übereinstimmung mit mindestens einem Steuersignal der Steuereinheit (10) steuert;
wobei die Steuereinheit (10) die Temperatur eines Patienten mit dem Aktuator (13) auf der Grundlage
- mindestens einer Soll-Körpertemperatur (Tb,target),
- der Ist-Temperatur (Tfa) und/oder der definierten Temperatur (Tfd) des Infusionsfluids,
- der Ist-Körpertemperatur (Tb),
- dem Ist-Durchfluss (FR) und
- dem mindestens einen den physiologischen Ist-Zustand des Patienten repräsentierenden Zusatzparameter (AP) steuert;
**dadurch gekennzeichnet, dass**
der mindestens eine Zusatzparameter (AP) mindestens den Parameter Herzfrequenzänderung aufweist,
wobei im Fall einer Soll-Körpertemperatur unter der Ist-Körpertemperatur (Tb) die Steuereinheit (10) geeignet ist, den Aktuator (13) so zu steuern, dass der Durchfluss (FR) reduziert und die Temperatur des Infusionsfluids reduziert wird, wodurch die Ist-Körpertemperatur (Tb) auf der Soll-Körpertemperatur gehalten wird, wenn der mindestens eine Zusatzparameter (AP) über einem ersten oberen Schwellwert liegt,
und/oder
der Durchfluss (FR) erhöht und die Temperatur des Infusionsfluids erhöht wird, wodurch die Ist-Körpertemperatur (Tb) auf der Soll-Körpertemperatur gehalten wird, wenn der mindestens eine Zusatzparameter (AP) unter einem ersten unteren Schwellwert liegt.

2. Vorrichtung (1) nach Anspruch 1, wobei die Vorrichtung (1) aufweist:
mindestens einen Fluidtemperatureingang (3), der mit der Steuereinheit (10) kommuniziert und geeignet ist, die Ist-Temperatur (Tfa) des Infusionsfluids und/oder einen definierten Wert für die Temperatur (Tfd) des Infusionsfluids zu empfangen.

3. Vorrichtung (1) nach einem der vorstehenden Ansprüche, wobei die Vorrichtung (1) mindestens einen Durchflusseingang (9) aufweist, der mit der Steuereinheit (10) kommuniziert und geeignet ist, den Ist-Durchfluss (FR) des Infusionsfluids zu empfangen, und/oder wobei die Vorrichtung (1) den Ist-Durchfluss (FR) des Infusionsfluids auf der Grundlage der Einstellungen des Aktuators (13) ableitet.

4. Vorrichtung (1) nach einem der vorstehenden Ansprüche, wobei die Steuereinheit (10) aufweist:
einen Speicher (11), der geeignet ist, Daten zu speichern, und/oder eine Datenverarbeitungseinheit (12), die geeignet ist, die Daten des Speichers (11) und/oder der Eingänge (3, 5, 7, 9) zu lesen und die Daten zu einem Steuersignal für den Aktuator (13) zu verarbeiten.

5. Vorrichtung (1) nach einem der vorstehenden Ansprüche, wobei der mindestens eine Zusatzparameter (AP) aufweist:
ein Aufnahmevermögen (AC), das das Vermögen des Patienten repräsentiert, zusätzliches Infusionsfluid aufzunehmen,
eine Reduktionsrate (RR), die das Vermögen des Patienten repräsentiert, das Infusionsfluid im Körper zu reduzieren, und/oder
einen Patientenzustandsindex (PSI).

6. Vorrichtung (1) nach einem der vorstehenden Ansprüche, wobei mindestens drei Zusatzparameter (AP) den physiologischen Ist-Zustand des Patienten repräsentieren und wobei die drei Zusatzparameter extravaskuläre Lungenflüssigkeitsmenge, zentraler Venendruck und Herzfrequenzänderung sind.

7. Vorrichtung (1) nach Anspruch 5, wobei das Aufnahmevermögen (AC) mindestens einer oder mehrere der Parameter: extravaskuläre Lungenflüssigkeitsmenge, zentraler Venendruck, intrakranieller Druck, intraabdominaler Druck, Kammerdruck, Herzfrequenz, Herzfrequenzvariabilität, Blutdruck, Arrhythmie, proBNP-Spiegel, Herzauswurffraktion, Ultraschall-Herzfüllungsstatus und ein Kapillarleckindex als Darstellung des Werts eines Kapillarlecksyndroms ist.

8. Vorrichtung (1) nach Anspruch 5, wobei die Reduktionsrate (RR) mindestens einer oder mehrere der Parameter: glomeruläre Filtrationsrate, Kreatinin-Clearance, Kreatininspiegel, Harnstoff-Clearance, Harnstoffspiegel, physiologische Abfallelimination und Eliminationsrate ist.

9. Vorrichtung (1) nach einem der vorstehenden Ansprüche, wöbei der mindestens eine Zusatzparameter (AP) mindestens einen oder mehrere der Parameter aufweist:
extravaskuläre Lungenflüssigkeitsmenge, zentraler Venendruck, Blutdruck, Kapillarleckindex als Darstellung des Werts eines Kapillarlecksyndroms, Eliminationsrate, periphere Perfusion, Splanchnikusperfusion, glomeruläre Filtrationsrate, Kreatinin-Clearance, Kreatininspiegel, Änderungsrate des Kreatininspiegels, Harnstoff-Clearance, Harnstoffspiegel, Änderungsrate des Harnstoffspiegels, intrakranieller Druck, Änderungsrate des intrakraniellen Drucks, Änderungsrate des intraabdominalen Drucks, intraabdominaler Druck, Änderungsrate des Kammerdrucks, Kammerdruck, Herzfrequenz, Herzfrequenzvariabilität, Arrhythmie, BNP-Spiegel, Änderungsrate des BNP-Spiegels, proBNP-Spiegel, NTproBNP-Spiegel, Änderungsrate des NTproBNP-Spiegels, Herzauswurffraktion, Ultraschall-Herzfüllungsstatus, physiologische Abfallelimination und Eliminationsänderungsrate.

10. Vorrichtung (1) nach einem der vorstehenden Ansprüche 5 bis 9, wobei die Datenverarbeitungseinheit (12) geeignet ist, den Patientenzustandsindex (PSI) zu berechnen, die Berechnung die Einschätzung, Priorisierung und/oder Gewichtung mindestens zweier oder mehrerer oder aller Patientenzustandsindexparameter (PSIP) aufweist, wobei der Patientenzustandsindex (PSI) mit mindestens einem oder mehreren der Patientenzustandsindexparameter (PSIP): periphere Perfusion, Splanchnikusperfusion, glomeruläre Filtrationsrate, Kreatinin-Clearance, Kreatininspiegel, Änderungsrate des Kreatininspiegels, Harnstoff-Clearance, Harnstoffspiegel, Änderungsrate des Harnstoffspiegels, intrakranieller Druck, Änderungsrate des intrakraniellen Drucks, Änderungsrate des intraabdominalen Drucks, intraabdominaler Druck, Änderungsrate des Kammerdrucks, Kammerdruck, Herzfrequenz, Herzfrequenzvariabilität, Arrhythmie, BNP-Spiegel, Änderungsrate des BNP-Spiegels, NTproBNP-Spiegel, Änderungsrate des NTproBNP-Spiegels, Herzauswurffraktion, Ultraschall-Herzfüllungsstatus, physiologische Abfallelimination und Eliminationsänderungsrate berechnet wird.

11. Vorrichtung (1) nach einem der vorstehenden Ansprüche 5 bis 10, wobei die Datenverarbeitungseinheit (12) geeignet ist, mindestens einen den physiologischen Ist-Zustand des Patienten repräsentierenden Zusatzparameter (AP) zu berechnen, und die Berechnung die Einschätzung, Priorisierung und/oder Gewichtung mindestens zweier der Parameter Aufnahmevermögen (AC), Reduktionsrate (RR) und/oder Patientenzustandsindex (PSI) aufweist.

12. Vorrichtung (1) nach einem der vorstehenden Ansprüche 5 bis 11, wobei die Datenverarbeitungseinheit (12) geeignet ist, mindestens einen den physiologischen Ist-Zustand des Patienten repräsentierenden Zusatzparameter (AP) zu berechnen, und die Berechnung die Einschätzung, Priorisierung und/oder Gewichtung mindestens zweier der Parameter Herzfrequenzänderung, zentraler Venenruck und/oder extravaskuläre Lungenflüssigkeitsmenge aufweist.

13. Vorrichtung (1) nach einem der vorstehenden Ansprüche 5 bis 12, wobei die Datenverarbeitungseinheit (12) geeignet ist, drei den physiologischen Ist-Zustand des Patienten repräsentierenden Zusatzparameter (AP) zu priorisieren, wobei die Zusatzparameter (AP) extravaskuläre Lungenflüssigkeitsmenge, zentraler Venendruck und Herzfrequenzänderung priorisiert werden und wobei die Infusion unterbrochen wird, wenn mindestens einer der Zusatzparameter (AP) über einem Schwellwert liegt.

14. Vorrichtung (1) nach einem der vorstehenden Ansprüche 6 bis 13, wobei Zusatzparameter (AP) zweiter Priorität Teil des Patientenzustandsindex (PSI) sind, und wobei die Infusion unterbrochen wird, wenn der Patientenzustandsindex (PSI) über einem Schwellwert liegt.

15. Vorrichtung (1) nach einem der vorstehenden Ansprüche, wobei der Benutzer die Parameter priorisiert, die mindestens ein den physiologischen Ist-Zustand des Patienten repräsentierenden Zusatzparameter (AP) sind und/oder Teil des Patientenzustandsindex (PSI) sind.

16. Vorrichtung (1) nach einem der vorstehenden Ansprüche 2 bis 15, wobei die Steuereinheit (10) über den Aktuator (13) mindestens zwei Infusionsparameter, darunter den Durchfluss (FR) und die Temperatur des Infusionsfluids, in Übereinstimmung mit der Soll-Körpertemperatur (Tb,target), vorzugsweise einer voreingestellten Soll-Körpertemperatur (Tb,target,preset), und/oder einem Soll-Körpertemperaturprofil steuert.

17. Vorrichtung (1) nach Anspruch 16, wobei das Soll-Körpertemperaturprofil ein benutzerdefiniertes Profil ist, das vorzugsweise auf mindestens einer voreingestellten Soll-Körpertemperatur (Tb,target,preset), einer Änderungsrate der Körpertemperatur (Tb) und/oder Soll-Durchfluss (FR) beruht.

18. Vorrichtung (1) nach einem der vorstehenden Ansprüche, wobei die Steuereinheit (10) über den Aktuator (13) die mindestens zwei Infusionsparameter innerhalb mindestens eines oberen und/oder unteren Schwellwerts der mindestens zwei Infusionsparameter steuert und/oder über den Aktuator (13) die Ist-Körpertemperatur (Tb) und/oder die Änderungsrate der Ist-Körpertemperatur (Tb) innerhalb mindestens eines oberen und/oder unteren Schwellwerts steuert.

19. Vorrichtung (1) nach einem der vorstehenden Ansprüche 16 bis 18, wobei die Steuereinheit (10) über den Aktuator (13) die mindestens zwei Infusionsparameter in Übereinstimmung mit mindestens einem oberen und/oder unteren Schwellwert des mindestens einen Zusatzparameters (AP) steuert.

20. Vorrichtung (1) nach einem der vorstehenden Ansprüche 16 bis 19, wobei die Steuereinheit (10) die Soll-Körpertemperatur (Tb,target) und/oder das Soll-Körpertemperaturprofil anpasst,
wenn die Ist-Soll-Körpertemperatur (Tb,target) und/oder das Soll-Körpertemperaturprofil nicht durch eine Einstellung des mindestens einen Infusionsparameters erreicht werden kann, die innerhalb des oberen und/oder unteren Schwellwerts für die mindestens zwei Infusionsparameter liegt, und die gewährleistet, dass der mindestens eine Zusatzparameter und/oder die Temperaturänderungsrate innerhalb des jeweiligen oberen und/oder unteren Schwellwerts liegen, und/oder wenn die Änderungsrate der Ist-Körpertemperatur (Tb) nicht innerhalb ihres oberen und/oder unteren Schwellwerts liegt.

21. Vorrichtung (1) nach einem der vorstehenden Ansprüche, wobei mindestens ein Sollwert und/oder mindestens ein Schwellwert für den mindestens einen Zusatzparameter (AP), für die mindestens zwei Infusionsparameter für die Ist-Körpertemperatur (Tb) und/oder für die Änderungsrate der Ist-Körpertemperatur (Tb) durch den Benutzer verwaltet werden und/oder durch den Hersteller eingestellte vordefinierte Daten sind.

22. Vorrichtung (1) nach einem der vorstehenden Ansprüche, wobei die Vorrichtung (1) geeignet ist, einen Alarm auszulösen und/oder eine Information oder unterschiedliche Statusinformationen anzuzeigen, wenn mindestens ein unterer oder oberer Schwellwert erreicht ist.

23. Vorrichtung (1) nach einem der vorstehenden Ansprüche, wobei der Fluidtemperatureingang (3), der Körpertemperatureingang (5), Zusatzeingang (7) und/oder der Durchflusseingang (9) aufweisen:
mindestens ein Sensorelement (4) und/oder
mindestens einen Verbinder (6) zum Verbinden mindestens eines Sensorelements (4) und/oder mindestens einer medizinischen Vorrichtung (8), wobei das mindestens eine Sensorelement (4) und/oder der mindestens eine Verbinder (6) geeignet sind, die Ist-Temperatur (Tfa) des Infusionsfluids, die Ist-Körpertemperatur (Tb) des Patienten, den mindestens einen den physiologischen Ist-Zustand des Patienten repräsentierenden Zusatzparameter (AP), den Ist-Durchfluss (FR) und/oder jeden anderen Parameter zu empfangen, zu messen und/oder zu verarbeiten.

24. Vorrichtung (1) nach einem der vorstehenden Ansprüche, wobei der mindestens eine Aktuator (13) in Fluidkommunikation mit der Zufuhr (2) steht und/oder wobei die Zufuhr (2) verbindbar ist und/oder mindestens ein Reservoir (17) aufweist, wobei das Reservoir (17) vorzugsweise ein Infusionsbeutel ist.

25. Vorrichtung (1) nach Anspruch 24, wobei das Reservoir (17) verbindbar ist und/ oder mindestens eine Temperaturreguliervorrichtung (18) und/oder mindestens einen Zufuhrtemperatursensor (21) aufweist.

26. Vorrichtung (1) nach Anspruch 25, wobei die Temperaturreguliervorrichtung (18) und/oder der Zufuhrtemperatursensor (21) mit der Steuereinheit (10) in Kommunikation stehen.

27. Vorrichtung (1) nach einem der vorstehenden Ansprüche, wobei die definierte Temperatur (Tfd) des Infusionsfluids durch einen Benutzer eingegeben oder durch eine externe Vorrichtung erhalten wird, vorzugsweise durch die Temperaturreguliervorrichtung (18) und/oder den Zufuhrtemperatursensor (21) erhalten wird.

28. Vorrichtung (1) nach einem der vorstehenden Ansprüche, wobei die Vorrichtung (1) mindestens einen Patientenverbinder (15) aufweist und/oder damit verbindbar ist, der den Aktuator (13) mit dem Patient verbindet.

## Revendications

1. Dispositif (1) pour la régulation ou l'aide à la régulation de la température d'un patient par perfusion d'un liquide, comprenant
au moins une alimentation (2) en liquide de perfusion ;
au moins une entrée de température corporelle (5) prévue pour recevoir la température corporelle effective (Tb) du patient ;
au moins une entrée additionnelle (7) prévue pour recevoir au moins un paramètre additionnel (AP) indicateur de l'état physiologique effectif du patient ;
au moins une unité de commande (10) reliée à l'entrée de température corporelle (5) et à l'entrée additionnelle (7) ; et
au moins un actionneur (13) commandant au moins le débit effectif (FR) et la température effective du liquide de perfusion en fonction d'au moins un signal de commande de l'unité de commande (10) ;
l'unité de commande (10) commandant la température d'un patient avec l'actionneur (13) sur la base
- d'au moins une température corporelle de consigne (Tb, consigne)
- de la température effective (Tfa) et/ou de la température définie (Tfd) du liquide de perfusion,
- de la température corporelle effective (Tb),
- du débit effectif (FR), et
- du ou des paramètres additionnels (AP) indicateurs de l'état physiologique effectif du patient ;
**caractérisé**
**en ce que** le ou les paramètres additionnels (AP) comprenant au moins le paramètre de variation du rythme cardiaque ;
**en ce qu'**en cas de température corporelle de consigne inférieure à la température corporelle effective (Tb), l'unité de commande (10) est prévue pour commander l'actionneur (13) de manière à
réduire le débit (FR) et à diminuer la température du liquide de perfusion en maintenant ainsi la température corporelle effective (Tb) à la température corporelle de consigne si le ou les paramètres additionnels (AP) dépassent une première valeur limite supérieure, et/ou
à augmenter le débit (FR) et à élever la température du liquide de perfusion en maintenant ainsi la température corporelle effective (Tb) à la température corporelle de consigne si le ou les paramètres additionnels (AP) sont inférieurs à une première valeur limite inférieure.

2. Dispositif (1) selon la revendication 1, où ledit dispositif (1) comprend au moins une entrée de température de liquide (3) reliée à l'unité de commande (10) et prévue pour recevoir la température effective (Tfa) du liquide de perfusion, et/ou une valeur définie pour la température (Tfd) du liquide de perfusion.

3. Dispositif (1) selon l'une des revendications précédentes, où ledit dispositif (1) comprend au moins une entrée de débit (9) reliée à l'unité de commande (10) et prévue pour recevoir le débit effectif (FR) du liquide de perfusion, et/ou où ledit dispositif (1) déduit le débit effectif (FR) du liquide de perfusion sur la base des réglages de l'actionneur (13).

4. Dispositif (1) selon l'une des revendications précédentes, où l'unité de commande (10) comprend :
une mémoire (11) prévue pour stocker des données, et/ou
une unité de traitement des données (12) prévue pour extraire les données de la mémoire (11) et/ou des entrées (3, 5, 7, 9), et traiter les données pour former un signal de commande pour l'actionneur (13).

5. Dispositif (1) selon l'une des revendications précédentes, où le ou les paramètres additionnels (AP) comprennent :
une capacité d'absorption (AC) indicatrice de la capacité du patient d'absorber du liquide de perfusion additionnel,
un taux de résorption (RR) indicateur de la capacité du patient de résorber le liquide de perfusion dans le corps, et/ou
un indice d'état du patient (PSI).

6. Dispositif (1) selon l'une des revendications précédentes, où au moins trois paramètres additionnels (AP) sont indicateurs de l'état physiologique effectif du patient, et où les trois paramètres additionnels sont : la quantité d'eau pulmonaire extravasculaire, la pression veineuse centrale et la variation du rythme cardiaque.

7. Dispositif (1) selon la revendication 5, où la capacité d'absorption (AC) est au moins un ou plusieurs des paramètres suivants : quantité d'eau pulmonaire extravasculaire, pression veineuse centrale, pression intracrânienne, pression intraabdominale, pression de compartiment, rythme cardiaque, variabilité du rythme cardiaque, tension artérielle, arythmie, taux de proBNP, fraction d'éjection cardiaque, état de saturation ultrasonique du coeur et un indice de fuite capillaire indicateur de la valeur d'un syndrome de fuite capillaire.

8. Dispositif (1) selon la revendication 5, où le taux de résorption (RR) est au moins un ou plusieurs des paramètres suivants :
taux de filtration glomérulaire, clairance de la créatinine, taux de créatinine, clairance de l'urée, taux d'urée, élimination des déchets physiologiques et taux d'élimination.

9. Dispositif (1) selon l'une des revendications précédentes, où le ou les paramètres additionnels (AP) comprennent au moins un ou plusieurs des paramètres suivants :
quantité d'eau pulmonaire extravasculaire, pression veineuse centrale, tension artérielle, indice de fuite capillaire indicateur de la valeur d'un syndrome de fuite capillaire, taux d'élimination,
perfusion périphérique, perfusion splanchnique, taux de filtration glomérulaire, clairance de la créatinine, taux de créatinine, variation du taux de créatinine, clairance de l'urée, taux d'urée, variation du taux d'urée, pression intracrânienne, variation de la pression intracrânienne, variation de la pression intraabdominale, pression intraabdominale, variation de la pression de compartiment, pression de compartiment, rythme cardiaque, variabilité du rythme cardiaque, arythmie, taux de BNP, variation du taux de BNP, taux de proBNP, taux de NTproBNP, variation du taux de NTproBNP, fraction d'éjection cardiaque, état de saturation ultrasonique du coeur, élimination des déchets physiologiques et variation du taux d'élimination.

10. Dispositif (1) selon l'une des revendications 5 à 9, où l'unité de traitement des données (12) est prévue pour calculer l'indice d'état du patient (PSI), et où le calcul comprend l'évaluation, la priorisation et/ou la pondération d'au moins deux, de plusieurs ou de tous les paramètres d'indice d'état du patient (PSIP), l'indice d'état du patient (PSI) étant calculé avec au moins un ou plusieurs des paramètres d'indice d'état du patient (PSIP) suivants : perfusion périphérique, perfusion splanchnique, taux de filtration glomérulaire, clairance de la créatinine, taux de créatinine, variation du taux de créatinine, clairance de l'urée, taux d'urée, variation du taux d'urée, pression intracrânienne, variation de la pression intracrânienne, variation de la pression intraabdominale, pression intraabdominale, variation de la pression de compartiment, pression de compartiment, rythme cardiaque, variabilité du rythme cardiaque, arythmie, taux de BNP, variation du taux de BNP, taux de NTproBNP, variation du taux de NTproBNP, fraction d'éjection cardiaque, état de saturation ultrasonique du coeur, élimination des déchets physiologiques et variation du taux d'élimination.

11. Dispositif (1) selon l'une des revendications 5 à 10, où l'unité de traitement des données (12) est prévue pour calculer au moins un paramètre additionnel (AP) indicateur de l'état physiologique effectif du patient, le calcul comprenant l'évaluation, la priorisation et/ou la pondération d'au moins deux des paramètres suivants : capacité d'absorption (AC), taux de résorption (RR) et/ou indice d'état du patient (PSI).

12. Dispositif (1) selon l'une des revendications 5 à 11, où l'unité de traitement des données (12) est prévue pour calculer au moins un paramètre additionnel (AP) indicateur de l'état physiologique effectif du patient, le calcul comprenant l'évaluation, la priorisation et/ou la pondération d'au moins deux des paramètres suivants : variation du rythme cardiaque, pression veineuse centrale et/ou quantité d'eau pulmonaire extravasculaire.

13. Dispositif (1) selon l'une des revendications 5 à 12, où l'unité de traitement des données (12) est prévue pour prioriser trois paramètres additionnels (AP) indicateurs de l'état physiologique effectif du patient, les paramètres additionnels (AP) quantité d'eau pulmonaire extravasculaire, pression veineuse centrale et variation du rythme cardiaque étant priorisés, et la perfusion étant interrompue si au moins un desdits paramètres additionnels (AP) dépasse une valeur limite.

14. Dispositif (1) selon l'une des revendications 6 à 13, où des paramètres additionnels (AP) de priorité secondaire appartiennent à l'indice d'état du patient (PSI), et où la perfusion est interrompue si l'indice d'état du patient (PSI) dépasse une valeur limite.

15. Dispositif (1) selon l'une des revendications précédentes, où l'utilisateur priorise les paramètres étant au moins un paramètre additionnel (AP) indicateur de l'état physiologique effectif du patient et/ou appartenant à l'indice d'état du patient (PSI).

16. Dispositif (1) selon l'une des revendications 2 à 15, où l'unité de commande (10) commande par l'intermédiaire de l'actionneur (13) au moins deux paramètres de perfusion, incluant le débit (FR) et la température du liquide de perfusion, en fonction de la température corporelle de consigne (Tb, consigne), préférentiellement une température corporelle de consigne par défaut (Tb, consigne, défaut) et/ou un profil de température corporelle de consigne.

17. Dispositif (1) selon la revendication 16, où le profil de température corporelle de consigne est un profil défini par l'utilisateur, préférentiellement sur la base d'au moins une température corporelle de consigne par défaut (Tb, consigne, défaut), d'une variation de température corporelle (Tb), et/ou d'un débit de consigne (FR).

18. Dispositif (1) selon l'une des revendications précédentes, où l'unité de commande (10) commande par l'intermédiaire de l'actionneur (13) les deux paramètres de perfusion au moins entre au moins une valeur limite supérieure et/ou une valeur limite inférieure des deux paramètres de perfusion au moins, et/ou commande par l'intermédiaire de l'actionneur (13) la température corporelle effective (Tb) et/ou la variation de la température corporelle effective (Tb) entre au moins une valeur limite supérieure et/ou une valeur limite inférieure.

19. Dispositif (1) selon l'une des revendications 16 à 18, où l'unité de commande (10) commande par l'intermédiaire de l'actionneur (13) les deux paramètres de perfusion au moins en fonction d'au moins une valeur limite supérieure et/ou une valeur limite inférieure du ou des paramètres additionnels (AP).

20. Dispositif (1) selon l'une des revendications 16 à 19, où l'unité de commande (10) adapte la température corporelle de consigne (Tb, consigne) et/ou le profil de température corporelle de consigne
si la température corporelle de consigne effective (Tb, consigne) et/ou le profil de température corporelle de consigne ne peuvent pas être obtenus par réglage d'au moins un paramètre de perfusion qui est compris entre la valeur limite supérieure et/ou la valeur limite inférieure pour les deux paramètres de perfusion au moins, et qui assure que le ou les paramètres additionnels et/ou la variation de température sont compris entre la valeur limite supérieure et/ou la valeur limite inférieure respectives, et/ou si la variation de la température corporelle effective (Tb) n'est pas comprise entre sa valeur limite supérieure et/ou sa valeur limite inférieure.

21. Dispositif (1) selon l'une des revendications précédentes, où au moins une valeur de consigne et/ou au moins une valeur limite pour le ou les paramètres additionnels (AP), pour les deux paramètres de perfusion au moins, pour la température corporelle effective (Tb) et/ou pour la variation de la température corporelle effective (Tb) sont administrées par l'utilisateur et/ou sont des données prédéfinies par le fabricant.

22. Dispositif (1) selon l'une des revendications précédentes, où ledit dispositif (1) est prévu pour déclencher une alarme et/ou pour afficher une information ou une information d'état différent si au moins une valeur limite supérieure ou une valeur limite inférieure sont atteintes.

23. Dispositif (1) selon l'une des revendications précédentes, où l'entrée de température de liquide (3), l'entrée de température corporelle (5), l'entrée additionnelle (7) et/ou l'entrée de débit (9) comprennent :
au moins un élément détecteur (4), et/ou
au moins un connecteur (6) pour la connexion d'au moins un élément détecteur (4) et/ou d'au moins un dispositif médical (8),
le ou les éléments détecteurs (4) et/ou au moins un connecteur (6) étant prévus pour recevoir, mesurer et/ou traiter la température effective (Tfa) du liquide de perfusion, là température corporelle effective (Tb) du patient, le ou les paramètres additionnels (AP) indicateurs de l'état physiologique effectif du patient, le débit effectif (FR) et/ou tout autre paramètre.

24. Dispositif (1) selon l'une des revendications précédentes, où le ou les actionneurs (13) sont en communication fluidique avec l'alimentation (2), et/ou où ladite alimentation (2) est raccordable et/ou comprend au moins un réservoir (17), ledit réservoir (17) étant préférentiellement une poche à perfusion.

25. Dispositif (1) selon la revendication 24, où le réservoir (17) est raccordable et/ou comprend au moins un dispositif de régulation de température (18), et/ou au moins un capteur de température d'alimentation (21).

26. Dispositif (1) selon la revendication 25, où le dispositif de régulation de température (18) et/ou le capteur de température d'alimentation (21) sont reliés à l'unité de commande (10).

27. Dispositif (1) selon l'une des revendications précédentes, où la température définie (Tfd) du liquide de perfusion est entrée par un utilisateur ou obtenue par un dispositif externe, préférentiellement obtenue par le dispositif de régulation de température (18) et/ou le capteur de température d'alimentation (21).

28. Dispositif (1) selon l'une des revendications précédentes, où ledit dispositif (1) comprend et/ou est raccordable à au moins un connecteur de patient (15) qui relie l'actionneur (13) au patient.
